(19) Europäisches Patentamt
European Patent Office
Office européen des brevets

(11) EP 1 510 575 A1

(12) **EUROPEAN PATENT APPLICATION**
published in accordance with Art. 158(3) EPC

(43) Date of publication:
**02.03.2005 Bulletin 2005/09**

(21) Application number: **03730703.0**

(22) Date of filing: **30.05.2003**

(51) Int Cl.[7]: **C12N 15/00**, C12Q 1/68, G01N 33/50

(86) International application number:
**PCT/JP2003/006804**

(87) International publication number:
**WO 2003/102178 (11.12.2003 Gazette 2003/50)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **31.05.2002 JP 2002158853**
**12.07.2002 JP 2002204143**
**19.07.2002 JP 2002211813**
**25.10.2002 JP 2002310862**
**19.11.2002 JP 2002335830**

(71) Applicant: **TAKARA BIO INC.**
**Otsu-shi, Shiga 520-2193 (JP)**

(72) Inventors:
- **KOBAYASHI, Eiji**
  **Otsu-shi, Shiga 520-2153 (JP)**
- **ENOKI, Tatsuji**
  **Kyotanabe-shi, Kyoto 610-0313 (JP)**
- **TANABE, Masashige**
  **Otsu-shi, Shiga 520-2133 (JP)**
- **UEDA, Yu**
  **Mizuho-shi, Gifu 501-0223 (JP)**
- **OKUDA, Shinji**
  **Koka-gun, Shiga 528-0041 (JP)**
- **SAGAWA, Hiroaki**
  **Kusatsu-shi, Shiga 525-0025 (JP)**
- **KATO, Ikunoshin**
  **Uji-shi, Kyoto 611-0028 (JP)**

(74) Representative: **Grund, Martin, Dr.**
**Dr. Volker Vossius,**
**Patentanwaltskanzlei,**
**Geibelstrasse 6**
**81679 München (DE)**

(54) **METHOD OF TYPING GENE POLYMORPHISMS**

(57) Accurate and highly reproducible means of detecting a base substitution mutation (for example, SNP), an insertion mutation or a deletion mutation with the use of a nucleic acid sample in a trace amount and a method of typing gene polymorphisms using the means.



Printed by Jouve, 75001 PARIS (FR)

**Description**

Technical Field

The present invention relates to a method for detecting a base substitution, an insertion mutation or a deletion mutation in a gene of interest and a kit for the method which are useful for typing of a gene.

Background Art

It is known that genetic codes contained in genomes of biological individuals belonging to the same species are not identical to each other, and there are differences in nucleotide sequences called polymorphisms. Ones in which one to tens of nucleotide(s) is (are) deleted or inserted, ones in which a specific nucleotide sequence is duplicated and the like are known as polymorphisms. One in which a single nucleotide is replaced by another nucleotide is called a single nucleotide polymorphism (SNP).

Conventional means of detecting SNPs are generally classified into ones based on hybridization, ones based on primer extension and ones utilizing substrate specificities of enzymes.

The presence of a base substitution is detected based on hybridization of a probe to a nucleic acid sample according to the hybridization method: According to this method, it is necessary to find a probe and hybridization conditions so that the hybridization is influenced by a difference in a single nucleotide. Therefore, it is difficult to establish a highly reproducible detection system.

A method for detecting a mutation using a cycle probe reaction (see, for example, United States Patent No. 5,660,988) exemplifies a conventional method. A method for detecting a mutation using the TaqMan method (see, for example, United States Patent Nos. 5,210,015 and 5,487,972) exemplifies another method. Further examples are as follows: methods in which a base substitution is detected based on the presence of a primer extension reaction using a primer whose 3' terminus anneals to a nucleotide portion for which a base substitution is to be detected (see, for example, United States Patent No. 5,137,806); methods in which a base substitution is detected based on the presence of a primer extension reaction using a primer in which the base substitution site to be detected is located at the second nucleotide from the 3' terminus (see, for example, WO 01/42498); and methods in which the presence of a mutation at the site of interest and the nucleotide at the site are determined by distinguishing a nucleotide incorporated into a primer using a primer whose 3' terminus anneals to a nucleotide adjacent on the 3' side to the nucleotide for which a base substitution is to be detected. Furthermore, methods in which a DNA ligase is used are known. According to this method, a base substitution is detected based on the presence of ligation, to an adjacent probe, of a probe whose terminal portion corresponds to the nucleotide portion for which a nucleotide substitution is to be detected. Further examples include methods in which an enzyme having an activity of recognizing and cleaving a specific structure in a double-stranded nucleic acid is utilized such as the invader method (see, for example, United States Patent No. 5,846,717).

The above-mentioned methods have problems as follows: the methods cannot be used to detect a trace amount of a target nucleic acid; the methods need to be carried out under strict temperature history conditions; the methods require strict control of annealing to a target nucleic acid; and the methods require an enzyme having a special property for the detection.

Then, a strand displacement-type nucleic acid amplification method such as the ICAN method (see, for example, WO 00/56877 and WO 02/16639) has been developed as a method for amplifying a target nucleic acid under isothermal conditions. Furthermore, the UCAN method (see, for example, WO 02/64833) has been developed as a method for typing a genetic polymorphism using a DNA-RNA-DNA-type chimeric oligonucleotide.

Objects of Invention

The main object of the present invention is to provide a means of detecting a base substitution mutation (e.g., an SNP), an insertion mutation or a deletion mutation with accuracy and excellent reproducibility using a trace amount of a nucleic acid sample, and to provide a method for typing a genetic polymorphism using the means.

Summary of Invention

A method that can be used to accurately detect various polymorphisms in genes such as base substitutions (e.g., SNPs), insertion mutations or deletion mutations, and with which the results can be obtained as intense signals is desired for solving the above-mentioned problems.

The present inventors have prepared a Nucleotide. The Nucleotide is capable annealing to a target nucleic acid for which a base substitution is to be detected. A DNA extension reaction with a DNA polymerase from the 3' terminus of the Nucleotide is not initiated if it is in an intact state. The cleavage of the Nucleotide with a nuclease is influenced by the nucleotide sequence of a template strand to which it anneals. Then, the present inventors have established a method that can be used to detect a genetic polymorphism in a target nucleic acid with accuracy and high sensitivity by using a combination of such a Nucleotide and a probe that can be used to specifically detect a target nucleic acid. The present inventors have further established a method that can be used to detect a genetic polymorphism with accuracy and high sensitivity by using a combination of a chimeric oligonucleotide primer, a strand displacement-type DNA polymerase, an RNase H and a probe that can be used to specifically detect a target nucleic acid. Thus, the present invention has been completed.

The first aspect of the present invention relates to a method for typing a genetic polymorphism at a specific nucleotide in a human cytochrome gene, G636A in the CYP 2C19 gene or T6235C in the CYP 1A1 gene, the method comprising:

(1) preparing a reaction mixture by mixing a nucleic acid as a template, deoxyribonucleotide triphosphates, a DNA polymerase having a strand displacement activity, at least one Nucleotide and an RNase H, wherein

(a) the Nucleotide is modified at the 3' terminus such that extension from the terminus by the action of a DNA polymerase does not take place; and
(b) the Nucleotide has a nucleotide sequence that is capable of annealing to a region containing the specific nucleotide in the human cytochrome gene; and

(2) incubating the reaction mixture for a time sufficient for generating a reaction product to extend a nucleic acid containing a region of arbitrary length that contains the specific nucleotide in the human cytochrome gene.

According to the first aspect, a Nucleotide having a nucleotide sequence of SEQ ID NO:14, 15, 21 or 22 or a nucleotide sequence complementary thereto can be preferably used as the Nucleotide. A primer having a nucleotide sequence of SEQ ID NO:16 or 23 may be further used.

The method of the first invention may comprise a step of detecting the nucleic acid extended in step (2) using a probe that is capable of hybridizing to the nucleic acid under stringent conditions. For example, a probe having a nucleotide sequence of SEQ ID NO:20 or 24 or a nucleotide sequence complementary thereto can be used in this step.

The second aspect of the present invention relates to a kit, which is used for the method of typing a genetic polymorphism in a human cytochrome gene of the first aspect, and which contains a Nucleotide having a nucleotide sequence of SEQ ID NO:14, 15, 21 or 22 or a nucleotide sequence complementary thereto.

The kit of the second aspect may further contain a primer having a nucleotide sequence of SEQ ID NO:16 or 23 and/or a probe having a nucleotide sequence of SEQ ID NO:20 or 24 or a nucleotide sequence complementary thereto.

The third aspect of the present invention relates to a method for typing a polymorphism in a human glutathione-S-transferase gene, the method comprising:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and
(b) incubating the reaction mixture for a time sufficient for generating a reaction product to amplify a target nucleic acid.

According to the third aspect, the human glutathione-S-transferase gene is exemplified by the GSTM1 gene or the GSTT1 gene. One having a nucleotide sequence of SEQ ID NO:6, 7, 8, 29, 30 or 31 or a nucleotide sequence complementary thereto can be preferably used as the chimeric oligonucleotide primer.

The method of the third aspect may further comprise a step of detecting the nucleic acid amplified in step (b) using a probe that is capable of hybridizing to the nucleic acid under stringent conditions. For example, probe having a nucleotide sequence of SEQ ID NO:9 or 32 or a nucleotide sequence complementary thereto can be used in this step.

The fourth aspect of the present invention relates to a kit, which is used for the method of typing a genetic polymorphism in a human glutathione-S-transferase gene of the third aspect, and which contains a Nucleotide having a nucleotide sequence of SEQ ID NO:6, 7, 8, 29, 30 or 31 or a nucleotide sequence complementary thereto.

The kit of the fourth aspect may further contain a probe having a nucleotide sequence of SEQ ID NO:9 or 32 or a nucleotide sequence complementary thereto.

The fifth aspect of the present invention relates to a method for typing a genetic polymorphism at a specific nucleotide in a human aldehyde dehydrogenase gene, Glu487Lys in the ALDH2 gene, the method comprising:

(1) preparing a reaction mixture by mixing a nucleic acid as a template, deoxyribonucleotide triphosphates, a DNA polymerase having a strand displacement activity, at least one Nucleotide and an RNase H, wherein

(a) the Nucleotide is modified at the 3' terminus such that extension from the terminus by the action of a DNA polymerase does not take place; and
(b) the Nucleotide has a nucleotide sequence that is capable of annealing to a region containing the specific nucleotide in the human aldehyde dehydrogenase gene; and

(2) incubating the reaction mixture for a time sufficient for generating a reaction product to extend a nucleic acid containing a region of arbitrary length that contains the specific nucleotide in the human aldehyde dehydrogenase gene.

According to the fifth aspect, a Nucleotide having a nucleotide sequence of SEQ ID NO:25 or 26 or a nucleotide sequence complementary thereto can be preferably used as the Nucleotide. A primer having a nucleotide sequence of SEQ ID NO:27 may be further used.
The method of the fifth aspect may comprise a step of detecting the nucleic acid extended in step (2) using a probe that is capable of hybridizing to the nucleic acid under stringent conditions. For example, a probe having a nucleotide sequence of SEQ ID NO:28 or a nucleotide sequence complementary thereto can be used in this step.
The sixth aspect of the present invention relates to a kit, which is used for the method of typing a genetic polymorphism in a human aldehyde dehydrogenase gene of the fifth aspect, and which contains a Nucleotide having a nucleotide sequence of SEQ ID NO:25 or 26 or a nucleotide sequence complementary thereto.
The kit of the sixth aspect may further contain a primer having a nucleotide sequence of SEQ ID NO:27 and/or a probe having a nucleotide sequence of SEQ ID NO:28 or a nucleotide sequence complementary thereto.
The seventh aspect of the present invention relates to a method for typing genetic polymorphisms, wherein plural target nucleic acids are detected in parallel, the method comprising:

(1) preparing a reaction mixture by mixing nucleic acids as templates, deoxyribonucleotide triphosphates, a DNA polymerase having a strand displacement activity, at least two Nucleotides and an RNase H, wherein

(a) each Nucleotide is modified at the 3' terminus such that extension from the terminus by the action of a DNA polymerase does not take place; and
(b) each Nucleotide has a nucleotide sequence that is capable of annealing to a region containing a specific nucleotide in the plural target nucleic acids; and

(2) incubating the reaction mixture for a time sufficient for generating reaction products to extend nucleic acids each containing a region of arbitrary length that contains the specific nucleotide.

The eighth aspect of the present invention relates to a method for typing genetic polymorphisms, wherein plural target nucleic acids are detected in parallel, the method comprising:

(a) preparing a reaction mixture by mixing nucleic acids as templates, deoxyribonucleotide triphosphates, a DNA polymerase having a strand displacement activity, at least two primers and an RNase H, wherein each primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and
(b) incubating the reaction mixture for a time sufficient for generating reaction products to amplify target nucleic acids.

The ninth aspect of the present invention relates to a method for typing genetic polymorphisms, wherein plural target nucleic acids are detected in parallel, the method comprising:

(A) detecting at least one target nucleic acid according to a method comprising:

(1) preparing a reaction mixture by mixing a nucleic acid as a template, deoxyribonucleotide triphosphates, a DNA polymerase having a strand displacement activity, at least one Nucleotide and an RNase H, wherein

(a) the Nucleotide is modified at the 3' terminus such that extension from the terminus by the action of a DNA polymerase does not take place; and
(b) the Nucleotide has a nucleotide sequence that is capable of annealing to a region containing a specific nucleotide in the plural target nucleic acids; and

(2) incubating the reaction mixture for a time sufficient for generating a reaction product to extend a nucleic acid containing a region of arbitrary length that contains the specific nucleotide; and

(B) detecting a target nucleic acid that is different from the target nucleic acid in (A) according to a method comprising:

(a) preparing a reaction mixture by mixing a nucleic acid as a template, deoxyribonucleotide triphosphates, a DNA polymerase having a strand displacement activity, at least two primers and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and
(b) incubating the reaction mixture for a time sufficient for generating a reaction product to amplify a target nucleic acid.

According to the seventh to ninth aspects, at least one of the target nucleic acids may be a nucleic acid as an internal standard. The nucleic acid as an internal standard may be detected using at least two primers selected from the group consisting of primers having nucleotide sequences of SEQ ID NOS:35-42. The nucleic acid as an internal standard may be detected further using a probe having a nucleotide sequence of SEQ ID NO:43 or 44 or a nucleotide sequence complementary thereto.
The tenth aspect of the present invention relates to a kit for the method of typing genetic polymorphisms of the seventh to ninth aspects.
The kit of the tenth aspect may contain at least two primers selected from the group consisting of primers having nucleotide sequences of SEQ ID NOS:35-42. It may further contain a probe having a nucleotide sequence of SEQ ID NO:43 or 44 or a nucleotide sequence complementary thereto.

Brief Description of Drawings

Figure 1 is a photograph that shows the results of detection of a deletion mutation in a human gene according to the detection method of the present invention.
Figure 2 is a photograph that shows the results of detection of a deletion mutation in a human gene according to the detection method of the present invention.
Figure 3 is a figure that shows the results of detection of a deletion mutation in a human gene according to the detection method of the present invention.
Figure 4 is a photograph that shows the results of detection of a base substitution in a human gene according to the method for detecting a base substitution of the present invention.
Figure 5 is a graph that shows the results of detection of a base substitution in a human gene according to the method for detecting a base substitution of the present invention.
Figure 6 is a photograph that shows the results of detection of a base substitution in a human gene according to the method for detecting a base substitution of the present invention.
Figure 7 is a figure that shows the results of detection of a base substitution in a human gene according to the method for detecting a base substitution of the present invention.
Figure 8 is a figure that shows the results of detection of a base substitution in a human gene according to the method for detecting a base substitution of the present invention.
Figure 9 is a photograph that shows the results of detection of a deletion mutation in a human gene according to the detection method of the present invention.
Figure 10 is a photograph that shows the results of detection of a deletion mutation in a human gene according to the detection method of the present invention.
Figure 11 is a figure that shows the results of detection of a deletion mutation in a human gene according to the detection method of the present invention.

Figure 12 is a graph that shows the results of detection of the human β-globin gene according to the detection method of the present invention.

Figure 13 is a figure that shows the results of detection of the human β-globin gene according to the detection method of the present invention.

Figure 14 is a figure that shows the results of detection of the human β-globin gene according to the detection method of the present invention.

Figure 15 is a figure that shows the results of detection of a deletion mutation in a human gene according to the detection method of the present invention.

Figure 16 is a figure that shows the results of detection of a deletion mutation in a human gene according to the detection method of the present invention.

Figure 17 is a figure that shows the results of detection of a deletion mutation in a human gene according to the detection method of the present invention.

Figure 18 is a figure that shows the results of detection of a deletion mutation in a human gene according to the detection method of the present invention.

Figure 19 is a figure that shows the results of typing of a human genetic polymorphism according to the detection method of the present invention.

Figure 20 is a figure that shows the results of detection of a deletion mutation in a human gene according to the detection method of the present invention.

Detailed Description of the Invention

As used herein, "a genetic polymorphism" refers to a difference in a nucleotide sequence of a gene among individuals in a population of the same species of an organism. The difference in a nucleotide sequence that constitutes a genetic polymorphism is not limited to a specific form. Various types such as "a base substitution", "a deletion mutation" and "an insertion mutation" as described below are included. The difference in genetic information is also called a variation.

As used herein, "a base substitution" refers to replacement of a nucleotide at a specific site in a nucleic acid by another nucleotide. There is no specific limitation concerning the number of substituted nucleotides in "the base substitution" according to the present invention. One or more nucleotide(s) may be substituted. A substitution observed for a single nucleotide in a nucleotide sequence is called "a single nucleotide polymorphism (SNP)". "The base substitutions" according to the present invention also include a base substitution artificially introduced into a nucleic acid.

As used herein, "a deletion mutation" means that a portion of a nucleotide sequence at a specific site in a nucleic acid is deleted. The deleted nucleotide sequence may consist of a single nucleotide or plural nucleotides. The deletion of a nucleotide sequence may occur at plural points at a specific site in a nucleic acid. "The deletion mutations" include deletion of a specific region of a gene (e.g., a region of an exon and/or an intron) and deletion of an entire gene. "The deletion mutations" according to the present invention also include deletion of a nucleotide sequence artificially introduced into a nucleic acid.

As used herein, "an insertion mutation" means that a nucleotide sequence is inserted at a specific site in a nucleic acid. The inserted nucleotide sequence may consist of a single nucleotide or plural nucleotides and may be of any chain length. The insertion of a nucleotide sequence may occur at plural points at a specific site in a nucleic acid. "The insertion mutations" according to the present invention also include insertion of a nucleotide sequence artificially introduced into a nucleic acid.

The present invention is suitable for detection of a genomic polymorphism or a variation, in particular, a base substitution (e.g., an SNP) in a gene, or detection of an insertion mutation and/or a deletion mutation at a specific site in a gene.

A single-stranded or double-stranded nucleic acid (RNA or DNA) can be used as a nucleic acid that contains a gene that serves as a subject for typing (a target nucleic acid) in the method for typing a genetic polymorphism of the present invention. Depending on the nuclease to be used, it may be difficult to use an RNA as a target nucleic acid. In this case, a base substitution in an RNA can be detected by preparing a cDNA using the RNA as a template and using the cDNA as a target nucleic acid.

According to the present invention, a sample. containing a target nucleic acid can be used for a typing reaction.

Any sample that may possibly contain a target nucleic acid such as a cell, a tissue (a biopsy sample, etc.), a whole blood, a serum, a cerebrospinal fluid, a seminal fluid, a saliva, a sputum, a urine, feces, a hair and a cell culture may be used without limitation. Although it is not intended to limit the present invention, the test sample may be subjected to the method of the present invention preferably after it is appropriately processed, for example, after it is converted into a form with which one can carry out a reaction with a DNA polymerase. Such processes include lysis of a cell as well as extraction and purification of a nucleic acid from a sample.

The present invention is described in detail below.

The method for typing a genetic polymorphism of the present invention is: (I) a method that is based on detection of the presence of a mismatch between a nucleotide sequence of a target nucleic acid and a Nucleotide that anneals thereto; or (II) a method in which deletion and/or insertion of a nucleotide sequence in a target nucleic acid is judged based on the chain length of a fragment amplified using a pair of primers or by determining whether or not amplification occurs. A suitable method may be selected depending on the type of the polymorphism to be typed.

(1) The method of typing a genetic polymorphism in a target nucleic acid of the present invention (I)

The method for typing a genetic polymorphism of the present invention (I) comprises:

(A) preparing a reaction mixture by mixing a nucleic acid as a template, deoxyribonucleotide triphosphates, a DNA polymerase having a strand displacement activity, at least one Nucleotide and an RNase H, wherein

(a) the Nucleotide is modified at the 3' terminus such that extension from the terminus by the action of a DNA polymerase does not take place; and
(b) the Nucleotide has a nucleotide sequence that is capable of annealing to a region containing a specific nucleotide at which a polymorphism may exist in a gene to be subjected to typing of a polymorphism; and

(B) incubating the reaction mixture for a time sufficient for generating a reaction product to extend a nucleic acid containing a region of arbitrary length that contains the specific nucleotide.

Optionally, a primer for amplifying a target nucleic acid may be further used in combination according to the typing method of the present invention.

The detection method of the present invention may optionally comprise a step of detecting the nucleic acid extended in step (B) using a probe that is capable of hybridizing to the nucleic acid under stringent conditions.

According to the method for typing a genetic polymorphism of the present invention, the presence of a base substitution is determined based on the presence of cleavage of a Nucleotide to be used and the presence of a DNA extension reaction subsequent to the cleavage. There is no specific limitation concerning the method for the determination, and known technique for nucleic acid analysis can be used. Examples of methods for determining the presence of a DNA extension reaction include the following: a method in which a generated extension product is separated for confirmation using gel electrophoresis (agarose gel, polyacrylamide gel, etc.) or capillary electrophoresis; and a method in which increase in length of an extension product is measured using mass spectrometry. A method in which incorporation of a nucleotide into an extension product is determined exemplifies another embodiment. In this method, one can have information about an amount of a synthesized extension product as an amount of a nucleotide triphosphate having an appropriate label incorporated into a macromolecular extension product. The amount of the generated extension product can be determined, for example, after separating the product from unreacted nucleotides by acid precipitation or gel electrophoresis. Furthermore, a method in which pyrophosphate generated upon a DNA extension reaction is detected by enzymatic means may be used.

According to the typing method of the present invention, a target nucleic acid of interest is amplified as a result of repeated extension reactions. The extension product may be further amplified using a known nucleic acid amplification reaction. Such an embodiment is useful in view of highly sensitive detection of a base substitution.

Various nucleic acid amplification methods in which a primer having a sequence complementary to a nucleic acid as a template is used can be used as the nucleic acid amplification reaction without limitation. For example, known amplification methods such as polymerase chain reaction (PCR, United States Patent Nos. 4,683,195, 4,683,202 and 4,800,159), strand displacement amplification (SDA, JP-B 7-114718), self-sustained sequence replication (3SR), nucleic acid sequence based amplification (NASBA, Japanese Patent No. 2650159), transcription-mediated amplification (TMA), and isothermal and chimeric primer-initiated amplification of nucleic acids (ICAN, WO 00/56877 and WO 02/16639) can be used. A genetic polymorphism in a target nucleic acid can be typed by using the Nucleotide according to the present invention as a primer for synthesis of a DNA complementary to a DNA strand as a template in such a method.

If the method for typing a genetic polymorphism of the present invention is carried out utilizing the above-mentioned nucleic acid amplification method, the Nucleotide according to the present invention is used as at least one of the primers used in the method, and a nuclease suitable for the Nucleotide is included in the reaction system.

According to the typing of a genetic polymorphism utilizing a nucleic acid amplification reaction as described above, the presence of a base substitution can be determined based on generation of a specific amplification product as a result of the reaction. Although it is not intended to limit the present invention, for example, gel electrophoresis, hybridization using a probe having a sequence complementary to the amplification product, a fluorescence polarization method utilizing a fluorescence labeled Nucleotide, a cycle probe reaction method and the like can be used for the generated amplification product. In addition, detection reactions suitable for the respective gene amplification methods can also be utilized.

If a base substitution is to be analyzed using the detection method of the present invention at a genomic level, it may be possible to reduce the volume of the reaction system and to use a means of increasing the degree of integration in combination for analyzing a large number of nucleotide sequences. A microchip, a micro-capillary electrophoresis (CE) chip or a nanochip exemplifies such a system.

Any nucleic acid amplification reaction may be utilized in such a system as long as the DNA fragment of interest is amplified using the reaction. Although it is not intended to limit the present invention, for example, a method in which a nucleic acid can be amplified under isothermal conditions such as the ICAN method can be preferably used. The combination with such a method can simplify the system and is very preferably utilized for the above-mentioned integrated system. Furthermore, a more highly integrated system can be constructed utilizing the techniques according to the present invention. The PCR method may be used in combination in the method of the present invention.

The above-mentioned Nucleotide having a label according to the present invention can facilitates confirmation of the presence of a DNA extension reaction, and is useful for the method for typing a genetic polymorphism of the present invention. In this case, the presence of an extension reaction may be confirmed by detecting a labeled substance derived from the Nucleotide using a method suitable for the label as described above.

For example, if the Nucleotide according to the present invention to which a fluorescent substance is attached is to be used and the label is attached to a portion that is utilized as a primer, an extension product can be detected utilizing the fluorescence. If a label is attached to a portion 3' to the cleavage site for a nuclease in a Nucleotide, the presence of an extension reaction can be detected based on dissociation of a 3' fragment from the target nucleic acid, conversion of the fragment into a smaller molecule due to a 5'→3' exonuclease activity of a DNA polymerase or the like. A fluorescence polarization method is preferably utilized for such an embodiment that involves change in molecular weight of a fluorescence labeled Nucleotide.

If the Nucleotide according to the present invention which is labeled by attaching a fluorescent substance and a substance having an action of quenching fluorescence emitted from the fluorescent substance such that the fluorescence is not emitted is to be used, the fluorescence is emitted at the same time as the initiation of an extension reaction. Therefore, a genetic polymorphism can be very readily typed.

In the above-mentioned respective embodiments, by utilizing Nucleotides each having adenine (A), cytosine (C), guanine (G), thymine (T) or uracil (U) at a position corresponding to the site for which a base substitution is to be detected as well as a distinguishable different label, one can have information about the presence of a base substitution and the type of the substituted base at the same time.

It is possible to carry out detection while distinguishing the following three types in higher animals including humans using the method of the present invention: homozygote (homo-type) in which both chromosomes do not have a base substitution; homozygote (homo-type) in which base substitutions are present on both chromosomes; and heterozygote (hetero-type) in which only one of chromosomes has a base substitution. Thus, the method of the present invention is also useful for detection of a base substitution in such an allele.

The Nucleotide used in the method of the present invention has a nucleotide sequence that is capable of annealing to a region containing a site in a target nucleic acid for which a base substitution is to be detected. The Nucleotide does not function as a primer for DNA extension by a DNA polymerase if it is in an intact state, and it can function as a primer only if it is cleaved by a nuclease. There is no specific limitation concerning the length of the Nucleotide as long as it has the properties as described above. Both an oligonucleotide and a polynucleotide can be used according to the present invention. Usually, an oligonucleotide of 8 to 50 nucleotides, preferably 10 to 40 nucleotides, more preferably 12 to 30 nucleotides is used as the Nucleotide according to the present invention.

The Nucleotide according to the present invention is usually an oligonucleotide containing deoxyribonucleotides. Optionally, it may contain a ribonucleotide, or an analog or a derivative (modification) of a nucleotide. For example, a nucleotide analog having a base such as inosine or 7-deazaguanine as its base moiety or a nucleotide analog having a ribose derivative can be used as a nucleotide analog. Examples of modified nucleotides include an ($\alpha$-S) nucleotide in which the oxygen atom attached to the phosphate group is replaced by a sulfur atom, and a nucleotide to which a labeled compound is attached. Furthermore, the Nucleotide according to the present invention may contain a peptide nucleic acid (PNA) (Nature, 365:566-568 (1993)). Although it is not intended to limit the present invention, the nucleotide analog or derivative is preferably incorporated at a site at which the incorporation does not influence the action of a nuclease to be used. Incorporation of a nucleotide analog into the Nucleotide according to the present invention is effective in view of suppression of higher order structure formation of the Nucleotide itself and stabilization of annealing of the Nucleotide to a target nucleic acid. Thus, the Nucleotide may contain a nucleotide analog and/or a modified nucleotide as long as the function as the Nucleotide that can be used in the method for typing a genetic polymorphism of the present invention is retained. The specificity of typing can be improved by including a modified nucleotide in the Nucleotide according to the present invention and/or by appropriately adjusting the reaction temperature.

The Nucleotide used according to the present invention has the following properties for typing of a polymorphism at a specific nucleotide in a target nucleic acid:

(A) the Nucleotide is modified at the 3' terminus such that extension from the terminus by the action of a DNA polymerase does not take place;

(B) the Nucleotide has a nucleotide sequence that is capable of annealing' to a region containing a specific base in the target nucleic acid; and

(C) the Nucleotide contains a sequence in which if there is a mismatch (or if there is no mismatch) between the specific nucleotide and a nucleotide corresponding to the specific nucleotide (i.e., that forms a hydrogen bond between the specific nucleotide) in the Nucleotide in a complex composed of the Nucleotide and the target nucleic acid, the Nucleotide is not cleaved with a nuclease, and if there is no mismatch (or if there is a mismatch) between the specific nucleotide and a nucleotide corresponding to the specific nucleotide in the Nucleotide, the Nucleotide is cleaved with a nuclease to generate a new 3' terminus.

A fragment of a 5' portion of the Nucleotide cleaved with a nuclease can remain annealed to a target nucleic acid. Since a hydroxyl group exists at the 3-position of ribose or deoxyribose at the 3' terminus of the fragment of the 5' portion of the Nucleotide, a DNA can be extended from the terminus by a DNA polymerase. Thus, the Nucleotide functions as a precursor of a primer if it has a nucleotide sequence that is cleavable with a nuclease.

As described above, the Nucleotide according to the present invention is modified at the 3' terminus such that it cannot be utilized for a DNA extension reaction with a DNA polymerase. There is no specific limitation concerning the means of modification as long as the above-mentioned objects can be achieved. Examples thereof include addition, at the 3' terminus, of a dideoxy nucleotide, a nucleotide modified at the hydroxyl group at the 3-position of ribose, or a nucleotide with modification that interferes with extension by the action of a DNA polymerase due to steric hindrance. Alkylation or other known modification methods can be utilized as a method for modifying the hydroxyl group at the 3-position of ribose of a nucleotide. For example, a DNA extension reaction can be prevented by aminoalkylation.

The Nucleotide has a nucleotide sequence that is capable of annealing, under conditions used, to a region in a target nucleic acid for which a polymorphism is to be studied. The Nucleotide has a sequence that is substantially complementary to a target nucleic acid, and need not have a nucleotide sequence completely complementary to the target nucleic acid as long as the detection of a substitution at the nucleotide of interest is not disturbed.

When the Nucleotide is annealed to a target nucleic acid and incubated in the presence of an appropriate nuclease and an appropriate DNA polymerase, cleavage of the Nucleotide is influenced by the presence of a base substitution in a target nucleic acid, that is, the presence of a mismatched site in a double-stranded nucleic acid formed by annealing of the Nucleotide to a target nucleic acid. DNA extension using the target nucleic acid as a template takes place only if the Nucleotide is cleaved to generate a new 3' terminus. Therefore, one can have information about the presence of a mismatch, or the presence of a base substitution based on the presence of DNA extension.

According to the present invention, it is possible to prepare the Nucleotide such that a mismatch is generated if there is a base substitution to be detected, and it is also possible to prepare the Nucleotide such that a mismatch is not generated if there is a base substitution. Furthermore, one can have information about the presence of a base substitution and the type of the substituted nucleotide at the same time as follows: four types of Nucleotides each having one of four types of nucleotides placed at a position corresponding to the nucleotide of interest are prepared and used; and the type of the nucleotide contained in the primer that results in extension is then examined.

As described above, the Nucleotide used in the present invention is converted into a primer that is capable of DNA extension as a result of cleavage with a nuclease. The portion of the Nucleotide 5' to the cleavage site for the nuclease functions as a primer for DNA extension. There is no specific limitation concerning the nuclease as long as it cleaves (or does not cleave) the Nucleotide depending on the presence of a mismatch in a double-stranded nucleic acid formed as a result of annealing of the Nucleotide to a target nucleic acid. Examples thereof include a ribonuclease H, a restriction enzyme and a mismatch-specific nuclease.

A ribonuclease H (RNase H) is an enzyme that recognizes a double-stranded nucleic acid composed of a DNA and an RNA and selectively cleaves the RNA strand. A Nucleotide that is cleaved with a ribonuclease H only if there is no mismatch can be prepared by placing a ribonucleotide at a site in the Nucleotide according to the present invention corresponding to the nucleotide for which a substitution is to be detected.

There is no specific limitation concerning the ribonuclease to be used according to the present invention as long as it has an activity of recognizing a double-stranded nucleic acid composed of the Nucleotide according to the present invention containing a ribonucleotide and a DNA complementary thereto and selectively cleaving at the ribonucleotide portion. A ribonuclease H can be preferably used as the enzyme. For example, a ribonuclease H from *Bacillus caldotenax*, *Pyrococcus furiosus*, *Pyrococcus horikoshii*, *Thermococcus litoralis*, *Thermotoga maritima*, *Archaeoglobus fulgidus* or *Methanococcus jannashi* can be used.

A restriction enzyme is an enzyme that recognizes a specific nucleotide sequence (of 4 to 8 nucleotides) in a DNA and cleaves at a position within or around the sequence. If the nucleotide portion for which a substitution is to be detected overlaps with a recognition sequence for a restriction enzyme, a Nucleotide prepared to include the sequence can be used for detection of a base substitution. If a mismatch is generated between a Nucleotide and a target nucleic acid, cleavage with a restriction enzyme does not take place. One can have information about the presence of the base substitution based on the results. If such a Nucleotide is to be used, it is necessary to make the target nucleic acid insusceptible to cleavage with the restriction enzyme. It is possible to confer resistance to the restriction enzyme specifically to the target nucleic acid, for example, by methylating the specific nucleotides using a modification methylase corresponding to the restriction enzyme to be used.

An enzyme that recognizes and cleaves a mismatch between a target nucleic acid and a Nucleotide unlike the above-mentioned two types of nucleases may be used. Mut H or the like may be used as such an enzyme.

The Nucleotide used according to the present invention is cleaved with the nuclease, a new 3' terminus is generated, and DNA extension is then initiated from the terminus. There is no specific limitation concerning the DNA polymerase used in this step as long as it is capable of DNA extension from the 3' terminus of a primer depending on the sequence of the DNA as a template. Examples thereof include *Escherichia coli* DNA polymerase I, Klenow fragment, T7 DNA polymerase, DNA polymerases from thermophilic bacteria belonging to genus *Bacillus* (Bst DNA polymerase, Bca DNA polymerase), DNA polymerases from bacteria belonging to genus *Thermus* (Taq DNA polymerase, etc.) and $\alpha$-type DNA polymerases from thermophilic archaebacteria (Pfu DNA polymerase, etc.).

If the Nucleotide according to the present invention is to be used in combination with a gene amplification reaction, a DNA polymerase suitable for the gene amplification reaction is selected and used.

According to the present invention, a fragment of a 3' portion of the Nucleotide generated as a result of cleavage with a nuclease can remain annealed to a target nucleic acid if it is sufficiently long, although it' may be released from the target nucleic acid if it is short. If a DNA polymerase having a strand displacement activity is used, the fragment is dessociated from the target nucleic acid upon DNA extension using the DNA polymerase. If a DNA polymerase having a 5'$\rightarrow$3' exonuclease activity is used, the fragment is degraded by the DNA polymerase.

Although it is not intended to limit the present invention, for example, an oligonucleotide having a structure represented by the following general formula can be used as the Nucleotide according to the present invention in case where a ribonuclease H is used as a nuclease:

General formula: 5'-dNa-Nb-dNc-N'-3'

(dN: deoxyribonucleotide and/or nucleotide analog; N: unmodified ribonucleotide and/or modified ribonucleotide; N': a nucleotide modified such that extension by the action of a DNA polymerase does not take place; wherein some of dNs in dNa may be replaced by Ns).

The portion represented by Nb in the general formula contains a nucleotide corresponding to the nucleotide as the subject of typing of a genetic polymorphism. Furthermore, the Nucleotide may contain a nucleotide analog or a derivative (a modified nucleotide) as long as the function of the Nucleotide is not spoiled.

Although it is not intended to limit the present invention, for example, a in the general formula is an integer of 5 or more, preferably 6 or more, more preferably 8 or more. b is an integer of 1 or more. For example, b is 1-15, preferably 1-10, more preferably 1-7, most preferably 1-5. c may be 0 or an integer of 1 or more, preferably 0-5, more preferably 0-3.

An exemplary Nucleotide is one represented by the general formula wherein N' is a modified deoxyribonucleotide, a=8, b=1 to 3, c=0 to 3. The values for a, b and c may be adjusted such that the Nucleotide can be used in the method of the present invention.

Detection of a fragment of a 3' portion released from the Nucleotide according to the present invention as a result of cleavage with a nuclease or due to a product generated upon a DNA extension reaction subsequent to the cleavage (an extension product) can be facilitated and the presence of a genetic polymorphism can be conveniently confirmed by appropriately labeling the Nucleotide.

There is no specific limitation concerning the method for labeling a Nucleotide. For example, radioisotopes ($^{32}P$, etc.), dyes, fluorescent substances, luminescent substances, various ligands (biotin, digoxigenin, etc.) and enzymes can be used. The presence of a product derived from a labeled Nucleotide can be confirmed using a detection method suitable for the label. A ligand that cannot be directly detected may be used in combination with a ligand-binding substance having a detectable label. For example, a target nucleic acid can be detected with high sensitivity by using a product from a ligand-labeled Nucleotide in combination with an enzyme-labeled anti-ligand antibody and amplifying the signal.

Examples of embodiments of fluorescence labeled Nucleotides include a Nucleotide labeled with both a fluorescent substance and a substance having an action of quenching fluorescence emitted from the fluorescent substance with appropriate spacing. Such a primer does not emit fluorescence if it is in an intact state. However, it emits fluorescence if it is cleaved with a nuclease, and' the fluorescent substance and the quenching substance are placed at a distance. Since such a Nucleotide emits fluorescence at the same time as the initiation of a DNA extension reaction, a genetic polymorphism can be typed by directly observing a reaction mixture during a reaction.

According to the method of the present invention, a nucleic acid as a template may optionally be physically, chemically or enzymatically denatured beforehand. There is no specific limitation concerning the denaturation method, and any method can be preferably used. Examples thereof include the following: heat denaturation (heat treatment of a' solution of a template nucleic acid at 90°C or above); alkali denaturation (treatment in an alkali solution); and enzymatic treatment (treatment with helicase, RecA or the like).

Examples of genes to be subjected to the method for typing a genetic polymorphism of the present invention include a human cytochrome gene (e.g., G636A in the CYP 2C19 gene or T6235C in the CYP 1A1 gene) and a human aldehyde dehydrogenase gene (e.g., Glu487Lys in the ALDH2 gene).

According to the method for typing a genetic polymorphism of the present invention, typing may be carried out while simultaneously or independently detecting a nucleic acid as an internal control. The internal control can be utilized for determination of false negatives in the method of the present invention. The internal control may be either one that is the same as a primer for extending a gene of interest and results in an extension product, or one that is different from a primer for extending a gene of interest and results in an extension product. The internal control may be a gene that is originally contained in a test sample. Although it is not intended to limit the present invention, for example, a β-globin gene or the like can be preferably used. Alternatively, a test sample to which an artificially prepared nucleic acid that can function as an internal control is added may be subjected to the method of the present invention. The internal control can be detected using the same method as the method used for typing a genetic polymorphism of interest.

(2) Kit used for the method for typing a genetic polymorphism of the present invention (I)

The present invention provides a kit used for the above-mentioned method for typing a genetic polymorphism of the present invention (I). In one embodiment, the kit contains a Nucleotide that can be used in the method of the present invention. Although there is no specific limitation concerning the Nucleotide, for example a Nucleotide having a nucleotide sequence of SEQ ID NO:14, 15, 21 and/or 22 is preferable for typing of a genetic polymorphism in a human cytochrome gene, and a Nucleotide having a nucleotide sequence of SEQ ID NO:25 and/or 26 is preferable for typing of a genetic polymorphism in a human aldehyde dehydrogenase gene. It may contain a set of Nucleotides each containing one of four types of nucleotides which can be used to determine the presence of a base substitution and the type of the substituted nucleotide at the same time. Examples of the Nucleotide sets that can be preferably used for typing of a human cytochrome gene include, but are not limited to, a set of Nucleotides 'having nucleotide sequences of SEQ ID NOS:14 and 15; a set of Nucleotides having nucleotide sequences of SEQ ID NOS:21 and 22; a set of Nucleotides and a primer having nucleotide sequences of SEQ ID NOS:14-16; and a set of Nucleotides and a primer having nucleotide sequences of SEQ ID NOS:21-23. A set of Nucleotides having nucleotide sequences of SEQ ID NOS:25 and 26 or a set of Nucleotides and a primer having nucleotide sequences of SEQ ID NOS:25-27 can be preferably used for typing of a human aldehyde dehydrogenase gene. The kit may contain a probe for specific detection having a nucleotide sequence of SEQ ID NO:20 or 24 which can be used to identify a base substitution in a human cytochrome gene, or a probe for specific detection having a nucleotide sequence of SEQ ID NO:28 which can be used to identify a base substitution in a human aldehyde dehydrogenase gene. Furthermore, the kit may contain a nuclease suitable for the Nucleotide, a DNA polymerase, a substrate for the DNA polymerase (dNTP), a buffer suitable for the reaction and the like. Alternatively, the kit may contain a reagent for detection of a primer extension product. A kit containing a reagent for preparing a reaction mixture used for a nucleic acid amplification method is preferable as a kit for typing a genetic polymorphism to be used in combination with the nucleic acid amplification method.

The kit of the present invention may contain a primer or a probe for detecting an internal control. In addition, the kit may contain a nucleic acid as an internal control to be added to a test sample. Examples of the primers and the probes include, but are not limited to, a primer that is used for amplifying β-globin and has a nucleotide sequence selected from the group consisting of nucleotide sequences of SEQ ID NOS:35-42 and a probe that is used for detecting β-globin and has a nucleotide sequence of SEQ ID NO:43 or 44 or a nucleotide sequence complementary thereto.

(3) The method for typing a genetic polymorphism in a target nucleic acid of the present invention (II)

The method for typing a genetic polymorphism of the present invention (II) comprises:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and

(b) incubating the reaction mixture for a time sufficient for generating a reaction product to amplify a target nucleic acid.

Optionally, the typing method may comprise a step of detecting the nucleic acid amplified in step (b) using a probe that is capable of hybridizing to the nucleic acid under stringent conditions.

A chimeric oligonucleotide primer used in the method of the present invention is a chimeric oligonucleotide primer that has a nucleotide sequence substantially complementary to a part of the nucleotide sequence of a nucleic acid as a template. It can contribute to extension of a DNA strand under conditions used. Furthermore, a ribonucleotide is positioned at the 3' terminus or on the 3'-terminal side of the chimeric oligonucleotide primer.

The chimeric oligonucleotide primer used in the method of the present invention may contain one or more modified ribonucleotide. As used herein, a ribonucleotide may be an unmodified ribonucleotide and/or a modified ribonucleotide that can be positioned at the 3' terminus or on the 3'-terminal side of a chimeric oligonucleotide primer and that is recognized by or cleaved with an endonuclease. The ribonucleotides include both of the unmodified ribonucleotide and the modified ribonucleotide as described above. An unmodified ribonucleotide, a modified ribonucleotide or a combination thereof can be used for the chimeric oligonucleotide primer according to the present invention as long as it does not abolish the function of the primer. Examples of the modified ribonucleotides include, but are not limited to, an ($\alpha$-S) ribonucleotide in which the oxygen atom bound to the phosphate group is replaced by a sulfur atom, and a ribonucleotide in which the hydroxy group at the 2-position of the ribose is replaced by a methoxy group. Such a chimeric oligonucleotide primer containing a modified ribonucleotide can be produced by using, for example, an ($\alpha$-S) ribonucleotide triphosphate, which is prepared by a method using a sulfuration reaction reagent (Glen Research) as described in United States Patent No. 5,003,097, or a 2-OMe-RNA-CE phosphoramidite reagent (Glen Research).

A chimeric oligonucleotide primer that can be used in the amplification method according to the present invention may be designed to contain a modified ribonucleotide that confers resistance to the cleavage with an endonuclease. Such a primer is useful in that one can control the cleavage site with an endonuclease during amplification reaction steps.

One or two chimeric oligonucleotide primer(s) may be used in the method of the present invention depending on the desired form of a DNA fragment after amplification (single-stranded or double-stranded). Specifically, one chimeric oligonucleotide primer is used when a single-stranded DNA is desired, whereas two primers are used when a double-stranded DNA is desired.

There is no specific limitation concerning the length of the chimeric oligonucleotide primer used in the present invention as long as the primer contains a ribonucleotide at the 3' terminus or on the 3'-terminal side and can be used in the ICAN method.

For example, the length of the chimeric oligonucleotide primer that can be used is about 6 nucleotides to about 100 nucleotides, preferably about 10 nucleotides to about 50 nucleotides, more preferably about 12 nucleotides to about 40 nucleotides. It is preferable that the nucleotide sequence of the chimeric oligonucleotide is substantially complementary to a nucleic acid as a template such that it anneals to the nucleic acid as the template under reaction conditions used. The primer contains a sequence recognized by an endonuclease, which is used in a step as described below, at the 3' terminus or on the 3'-terminal side.

For example, an oligonucleotide having a structure represented by the following general formula can be used in the DNA synthesis method according to the present invention as a primer, although it is not intended to limit the present invention:

General formula: 5'-dNa-Nb-dNc-3'

(dN: deoxyribonucleotide and/or nucleotide analog; N: unmodified ribonucleotide and/or modified ribonucleotide, wherein some of dNs in dNa may be replaced by Ns, and the nucleotide at the 3' terminus may be modified such that extension from the terminus by the action of the DNA polymerase does not take place).

Although it is not intended to limit the present invention, for example, a in the general formula is an integer of 5 or more, preferably 6 or more, more preferably 8 or more. b is an integer of 1 or more. For example, b is 1-15; preferably 1-10, more preferably 1-7, most preferably 1-5. c may be 0 or an integer of 1 or more, preferably 0-5, more preferably 0-3.

Although it is not intended to limit the present invention, examples of the chimeric oligonucleotide primers used according to the present invention include one represented by the general formula wherein a=8; and b=1 and c=0; b=2 and c=0; b=3-5 and c=0; or b=1-3 and c=0-3. The values for a, b and c may be adjusted such that the chimeric oligonucleotide primer can be used in the method of the present invention.

Furthermore, the chimeric oligonucleotide primer used in the method of the present invention may contain nucleotide analog or other substances. That is, one or more nucleotide analog(s) can be contained in the chimeric oligonucleotide primer according to the present invention as long as the function of the primer for effecting a polymerization extension reaction from the 3' terminus by the action of a DNA polymerase is not abolished. Plural types of the nucleotide analogs can be used in combination. Examples of the nucleotide analogs that can be used include, but are not limited to, deoxyinosine nucleotide, deoxyuracil nucleotide, a deoxyribonucleotide analog having a modified base such as 7-deazaguanine, a nucleotide analog having a ribose derivative and the like. Furthermore, the chimeric oligonucleotide primers used in the present invention may contain deoxynucleotides, ribonucleotides or nucleotide analogs having various modifications such as addition of labeled compounds as long as they retain the functions as described above.

Incorporation of a nucleotide analog into a primer is effective for suppressing the formation of higher order structure of the primer itself and stabilization of annealing formation with the template. A ribonucleotide may be incorporated into a primer for the same purpose. Although it is not intended to limit the present invention, a modified ribonucleotide such as ($\alpha$-S) ribonucleotide can be preferably used in order to prevent the digestion of the primer by a non-specific endonuclease (RNase).

The chimeric oligonucleotide primer can be synthesized to have desired nucleotide sequence using a known method or a commercially available nucleic acid synthesis instrument.

In step (a) in the method of the present invention, if an RNA is used as a template, the reverse transcription reaction and the nucleic acid amplification reaction may be conducted in a single step. Although it is not intended to limit the present invention, for example, a combination of AMV RTase, MMLV RTase or RAV-2 RTase and Bca DNA polymerase can be preferably used as a combination of a reverse transcriptase and a strand displacement-type DNA polymerase.

The chain length of the target nucleic acid to be amplified according to the method of the present invention is not limited to a specific one. For example, a region of 200 bp or shorter, preferably 150 bp or shorter is effective for sensitive detection of the target nucleic acid. Typing of a genetic polymorphism can be carried out with high sensitivity by designing the chimeric oligonucleotide primers according to the present invention to result in the chain length to be amplified as described above.

In addition, a target nucleic acid can be detected with higher sensitivity even from a trace amount of a nucleic acid sample in the detection method of the present invention by using a reaction buffer containing Bicine, Tricine, HEPES, phosphate or tris as a buffering component and an annealing solution containing spermidine or propylenediamine. In this case, the endonuclease and the DNA polymerase to be used are not limited to specific ones. For example, a combination of an RNase H from *Escherichia coli*, a bacterium of genus *Pyrococcus* or a bacterium of genus *Archaeoglobus* and BcaBEST DNA polymerase (Takara Bio) is preferable. It is considered that the preferable units of the endonuclease and the DNA polymerase may vary depending on the types the enzymes. In such a case, the composition of the buffer and the amount of the enzymes added may be adjusted using the increase in detection sensitivity or the amount of amplification product as an index.

According to the present invention, dUTP may be incorporated as a substrate during amplification of a target nucleic acid. Thus, if dUTP is used as a substrate, it is possible to prevent false positives due to carry-over contamination of amplification products by degrading amplification products utilizing uracil N-glycosidase (UNG).

The presence of an insertion mutation and/or a deletion mutation can be judged according to the gene typing method of the present invention by determining the generation of an amplification product or the chain length of an amplification product in steps (a) and (b). One can decide to carry out the judgment based on the presence of an amplification product or the chain length of an amplification product by selecting the position of a chimeric oligonucleotide primer in the gene to be typed.

Known methods for detecting a nucleic acid can be used for the detection steps as described above. Examples of such methods include detection of a reaction product having a specific size by electrophoresis, and detection by hybridization with a probe. Furthermore, a detection method in which magnetic beads or the like are used in combination can be preferably used. Pyrophosphoric acid generated upon amplification of a target nucleic acid may be converted into an insoluble substance such as a magnesium salt to make the reaction mixture cloudy, and the turbidity may be measured. A fluorescent substance such as ethidium bromide is usually used in the detection by electrophoresis. The hybridization with a probe may be combined with the detection by electrophoresis. The probe may be labeled with a radioisotope or with a non-radioactive substance such as biotin or a fluorescent substance. Additionally, use of a labeled nucleotide in step (b) may facilitate the detection of amplification product into which the labeled nucleotide is incorporated, or may enhance the signal for detection utilizing the label. A fluorescence polarization method, a fluorescence resonance energy transition (FRET), a non-fluorescence resonance energy transition (Non-FRET), a method in which an electrode and deposition of a conductive substance are used in combination or the like can also be utilized for the detection. The target nucleic acid can be detected automatically or quantified by constructing a suitable detection system. In addition, detection with naked eyes by a hybrid chromatography method can be preferably used.

A ribonucleotide (RNA) probe, or a chimeric oligonucleotide probe composed of a ribonucleotide and a deoxyribonucleotide, labeled with two or more fluorescent substances positioned at a distance that results in a quenching state can be used in the detection method of the present invention. For example, a combination of 6-carboxyfluorescein (6-FAM) and N,N,N',N'-tetramethyl-6-carboxyrhodamine (TAMRA), which is a pair of labels for FRET, or a combination of 6-carboxyfluorescein (6-FAM) and 4-(4'-dimethylaminophenylazo)benzoic acid (DABCYL) , which is a pair of labels for Non-FRET, can be preferably used as fluorescent substances for labeling the probe.

The probe used in the present invention is not limited to specific one as long as it can hybridize to a target nucleic acid amplified by the nucleic acid amplification method according to the present invention under normal hybridization conditions. In view of specific detection of amplification product, a probe that hybridizes under conditions, for example, known to those skilled in the art as being stringent is preferable. The stringent hybridization conditions are described in, for example, T. Maniatis et al. (eds.), Molecular Cloning: A Laboratory Manual 2nd ed., 1989, Cold Spring Harbor Laboratory. Specifically, the stringent conditions refer to the following: incubation at a temperature about 25°C lower than the Tm of the probe to be used for 4 hours to overnight in 6 x SSC (1 x SSC: 0.15 M NaCl, 0.015 M sodium citrate, pH 7.0) containing 0.5% SDS, 5 x Denhardt's (0.1% bovine serum albumin (BSA), 0.1% polyvinylpyrrolidone, 0.1% Ficoll 400) and 100 µg/ml salmon sperm DNA. A probe having a label as described above may be used as the probe for facilitating the detection of the target nucleic acid.

The method for amplifying a nucleic acid under isothermal conditions according to the present invention does not require the use of equipment such as a thermal cycler. The number of primers used in the amplification method according to the present invention can be one or two, which is less than that used in a conventional method. Since reagents such as dNTPs used for PCR and the like can be applied to the method, the running cost can be reduced as compared with a conventional method. Therefore, the method can be preferably used, for example, in a field of genetic test in which the detection is routinely conducted. The method according to the present invention provides a greater amount of an amplification product in a shorter time than the PCR. Therefore, the method can be utilized as a convenient, rapid and highly sensitive method for detecting a gene.

One can convert the method for typing an insertion mutation and/or a deletion mutation of the present invention into a highly efficient analysis system that is suitable for large-scale processing by making the volume of the reaction system smaller and using a means of increasing degree of integration in combination as described above.

Also according to the method of the present invention, a nucleic acid as a template may optionally be physically, chemically or enzymatically denatured beforehand as described in (1) above.

Examples of subjects of the method for typing a genetic polymorphism of the present invention include, but are not limited to, a deletion mutation in a human glutathione-S-transferase gene (e.g., the GSTM1 gene or the GSTT1 gene). According to the detection method of the present invention, one or both of paired chimeric oligonucleotide primers may be located in the region of the deletion mutation. Also according to the method for typing a genetic polymorphism of the present invention, typing may be carried out while simultaneously or independently detecting a nucleic acid as an internal control as described in above for the method for typing a genetic polymorphism (1).

(4) Kit used for the method for typing a genetic polymorphism of the present invention (II)

The present invention provides a kit used for the method for typing a genetic polymorphism (II). In one embodiment, the kit contains a chimeric oligonucleotide primer that can be used in the method of the present invention. Although there is no specific limitation concerning the primer, for example, ones having nucleotide sequences of SEQ ID NOS:6-8 and 29-31 are preferable for detection of a deletion mutation in a human glutathione-S-transferase gene. The kit may contain a probe for specific detection that can be used to identify an insertion mutation and/or a deletion mutation. Although it is not intended to limit the present invention, for example, nucleotides having nucleotide sequences of SEQ ID NOS:9 and 32 can be preferably used as'such a probe. Furthermore, the kit may contain a nuclease suitable for the nucleotide, a DNA polymerase, substrates for the DNA polymerase (dNTPs), a buffer suitable for the reaction and the like. The kit may contain a reagent for detecting a primer extension product. A kit containing a reagent for preparing a reaction mixture used for a nucleic acid amplification method is preferable as a kit for typing a genetic polymorphism to be used in combination with the amplification method.

The kit of the present invention may contain a primer and a probe for detecting an internal control like the kit for the method for typing a genetic polymorphism (2).

(5) The method for typing a genetic polymorphism in plural target nucleic acids and the kit for the method of the present invention

The following exemplifies one embodiment of the method for typing genetic polymorphisms in plural target nucleic acids of the present invention: a method for typing genetic polymorphisms, wherein plural target nucleic acids are detected in parallel, the method comprising:

(1) preparing a reaction mixture by mixing a nucleic acids as templates, deoxyribonucleotide triphosphates, a DNA polymerase having a strand displacement activity, at least two Nucleotides and an RNase H, wherein

(a) each Nucleotide is modified at the 3' terminus such that extension from the terminus by the action of a DNA polymerase does not take place; and
(b) each Nucleotide has a nucleotide sequence that is capable of annealing to a region containing a specific nucleotide in the plural target nucleic acids; and

(2) incubating the reaction mixture for a time sufficient for generating reaction products to extend nucleic acids each containing a region of arbitrary length that contains the specific nucleotide.

The following exemplifies another embodiment: a method for typing genetic polymorphisms, wherein plural target nucleic acids are detected in parallel, the method comprising:

(a) preparing a reaction mixture by mixing nucleic acids as templates, deoxyribonucleotide triphosphates, a DNA polymerase having a strand displacement activity, at least two primers and an RNase H, wherein each primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and
(b) incubating the reaction mixture for a time sufficient for generating reaction products to amplify target nucleic acids.

The following exemplifies a still another embodiment: a method for typing genetic polymorphisms, wherein plural target nucleic acids are detected in parallel, the method comprising:

(A) detecting at least one target nucleic acid according to a method comprising:

(1) preparing a reaction mixture by mixing a nucleic acid as a template, deoxyribonucleotide triphosphates, a DNA polymerase having a strand displacement activity, at least one Nucleotide and an RNase H, wherein

(a) the Nucleotide is modified at the 3' terminus such that extension from the terminus by the action of a DNA polymerase does not take place; and
(b) the Nucleotide has a nucleotide sequence that is capable of annealing to a region containing a specific nucleotide in the plural target nucleic acids; and

(2) incubating the reaction mixture for a time sufficient for generating a reaction product to extend a nucleic acid containing a region of arbitrary length that contains the specific nucleotide; and

(B) detecting a target nucleic acid that is different from the target nucleic acid in (A) according to a method comprising:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and
(b) incubating the reaction mixture for a time sufficient for generating a reaction product to amplify a target nucleic acid.

According to the method for typing genetic polymorphisms in plural target nucleic acids of the present invention, there is no specific limitation concerning the target nucleic acid. In other words, any genes that serve as subjects for typing of genetic polymorphisms can be preferably used. The combination of genetic polymorphisms in target nucleic acids may be any combination of identical or distinct ones of various types such as "a base substitution", "a deletion mutation" and "an insertion mutation". Examples of the combinations of genetic polymorphisms in target nucleic acids include, but are not limited to, combinations of ones selected from the group consisting of human cytochrome genes CYP 2C19 and CYP 1A1, human glutathione-S-transferase genes GSTM1 and GSTT1, and human aldehyde dehydrogenase gene ALDH2.

For example, one as described in (1) to (4) above can be preferably used as a primer or a probe for detecting a genetic polymorphism.

According to the method of the present invention, the combination of target nucleic acids may be a combination of a gene having at least one of the above-mentioned types of genetic polymorphisms and another gene without a polymorphism. There is no specific limitation concerning the gene without a polymorphism. Furthermore, a nucleic acid as an internal control may be used in combination upon detection according to the present invention.

Specifically, at least one of the plural target nucleic acids may be a nucleic acid as an internal control. Although it is not intended to limit the present invention, for example, a β-globin gene can be preferably used as such a nucleic acid. Although it is not intended to limit the present invention, for example, at least two primers selected from the group consisting of primers having nucleotide sequences of SEQ ID NOS:35-42 can be used as primers for detecting the β-globin gene. Furthermore, the detection can be carried out using a probe having a nucleotide sequence of SEQ ID NO:43 or 44 or a nucleotide sequence complementary thereto. According to the typing method of the present invention, a target nucleic acid of interest is amplified as a result of repeated extension reactions. The extension product may be further amplified using a known nucleic acid amplification reaction.

Furthermore, the kit for the method of the present invention may contain a Nucleotide or a primer for detecting plural target nucleic acids. The kit may contain a probe for detecting an amplification product obtained according to the method of the present invention.

For example, if at least one of the plural target nucleic acids is a nucleic acid as an internal control, the β-globin gene, the kit may contain at least two primers selected from the group consisting of primers having nucleotide sequences of SEQ ID NOS:35-42, and it may contain a probe having a nucleotide sequence of SEQ ID NO:43 or 44 or a nucleotide sequence complementary thereto.

The method of the present invention can be used to detect a base substitution, an insertion mutation and a deletion mutation. Example of subject genes include, but are not limited to, a base substitution such as one in a human cytochrome gene, G636A (m2) in the CYP 2C19 gene or T6235C in the CYP 1C1 gene, or Glu487Lys in the aldehyde dehydrogenase 2 (ALDH2) gene, and a deletion mutation in a glutathione-S-transferase gene, GSTM1 or GSTT1. The method of the present invention can be suitably used to detect such a gene. Furthermore, the method of the present invention can be used as a method for typing a gene.

Examples

The following examples further illustrate the present invention in detail but are not to be construed to limit the scope thereof.

Referential Example 1: Cloning of RNase HII gene from *Archaeoglobus fulgidus*

(1) Preparation of genomic DNA from *Archaeoglobus fulgidus*

Cells of *Archaeoglobus fulgidus* (purchased from Deutsche Sammlung von Mikroorganismen und Zellkulturen GmbH; DSM4139) collected from 8 ml of a culture was suspended in 100 μl of 25% sucrose, 50 mM Tris-HCl (pH 8.0). 20 μl of 0.5 M EDTA and 10 μl of a 10 mg/ml lysozyme chloride (Nacalai Tesque) aqueous solution was added thereto. The mixture was reacted at 20°C for 1 hour. After reaction, 800 μl of a mixture containing 150 mM NaCl, 1 mM EDTA and 20 mM Tris-HCl (pH 8.0), 10 μl of 20 mg/ml proteinase K (Takara Bio) and 50 μl of a 10% sodium lauryl sulfate aqueous solution were added to the reaction mixture. The mixture was incubated at 37°C for 1 hour. After reaction, the mixture was subjected to phenol-chloroform extraction, ethanol precipitation and air-drying, and then dissolved in 50 μl of TE to obtain a genomic DNA solution.

(2) Cloning of RNase HII gene

The entire genomic sequence of *Archaeoglobus fulgidus* has been published [Klenk, H.P. et al., Nature, 390: 364-370 (1997)]. The existence of one gene encoding a homologue of RNase HII (AF0621) was known (SEQ ID NO: 1, http://www.tigr.org/tdb/CMR/btm/htmls/SplashPage.htlm).

Primers AfuNde (SEQ ID NO:2) and AfuBam (SEQ ID NO:3) were synthesized on the basis of the sequence of the AF0621 gene (SEQ ID NO:1).

A PCR was carried out using 30 ng of the *Archaeoglobus fulgidus* genomic DNA prepared in Referential Example 1-(1) as a template, and 20 pmol of AfuNde and 20 pmol of AfuBam as primers in a volume of 100 µl. Pyrobest DNA polymerase (Takara Bio) was used as a DNA polymerase for the PCR according to the attached protocol. The PCR was carried out as follows: 40 cycles of 94°C for 30 seconds, 55°C for 30 seconds and 72°C for 1 minute. An amplified DNA fragment of about 0.6 kb was digested with NdeI and BamHI (both from Takara Bio). The resulting DNA fragment was inserted between the NdeI site and the BamHI site in a plasmid vector pTV119Nd (a plasmid in which the NcoI site in pTV119N is converted into a NdeI site) to make a plasmid pAFU204.

(3) Determination of nucleotide sequence of DNA fragment containing RNase HII gene

The nucleotide sequence of the DNA fragment inserted into pAFU204 obtained in Referential Example 1-(2) was determined according to a dideoxy method.

Analysis of the determined nucleotide sequence revealed an open reading frame presumably encoding RNase HII. The nucleotide sequence of the open reading frame is shown in SEQ ID NO:4. The amino acid sequence of RNase HII deduced from the nucleotide sequence is shown in SEQ ID NO:5.

*Escherichia coli* JM109 transformed with the plasmid pAFU204 is designated and indicated as *Escherichia coli* JM109/pAFU204, and deposited on February 22, 2001 at International Patent Organism Depositary, National Institute of Advanced Industrial Science and Technology, AIST Tsukuba Central 6, 1-1, Higashi 1-Chome, Tsukuba-shi, Ibaraki-ken 305-8566, Japan under accession number FERM P-18221 and at International Patent Organism Depositary, National Institute of Advanced Industrial Science and Technology under accession number FERM BP-7691 (date of transmission to international depositary authority: August 2, 2001).

(4) Preparation of purified RNase HII preparation

*Escherichia coli* JM109 was transformed with pAFU204 obtained in Referential Example 1-(2). The resulting *Escherichia coli* JM109 harboring pAFU204 was inoculated into 2 L of LB medium containing 100 µg/ml of ampicillin and cultured with shaking at 37°C for 16 hours. After cultivation, cells collected by centrifugation were suspended in 37.1 ml of a sonication buffer [50 mM Tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 70°C for 15 minutes. It was then centrifuged at 12000 rpm for 10 minutes again to collect a supernatant. Thus, 40.3 ml of a heated supernatant was obtained.

The heated supernatant was subjected to RESOURSE Q column (Amersham Pharmacia Biotech) equilibrated with Buffer A [50 mM Tris-HCl (pH 8.0), 1 mM EDTA] and chromatographed using FPLC system (Amersham Pharmacia Biotech). As a result, RNase HII flowed through the RESOURSE Q column.

The flow-through RNase HII fraction was subjected to RESOURSE S column (Amersham Pharmacia Biotech) equilibrated with Buffer A and chromatographed using FPLC system (Amersham Pharmacia Biotech). As a result, RNase HII flowed through the RESOURSE S column.

40.0 ml of the flow-through RNase HII fraction was subjected to three rounds of dialysis against 2 L of Buffer B (50 mM Tris-HCl (pH 7.0), 1 mM EDTA) containing 50 mM NaCl for 2 hours. 40.2 ml of the dialyzed enzyme solution was subjected to HiTrap-heparin column (Amersham Pharmacia Biotech) equilibrated with Buffer B containing 50 mM NaCl and eluted with a linear gradient of 50 to 550 mM NaCl using FPLC system. As a result, a fraction containing RNase HII eluted with about 240 mM NaCl was obtained.

7.8 ml of the RNase HII fraction was concentrated by ultrafiltration using Centricon-10 (Amicon). Four portions separated from about 600 µl of the concentrate were subjected to Superose 6 gel filtration column (Amersham Pharmacia Biotech) equilibrated with 50 mM Tris-HCl (pH 7.0) containing 100 mM NaCl and 0.1 mM EDTA and eluted with the same buffer. As a result, RNase HII was eluted at a position corresponding to a molecular weight of 30.0 kilodalton. This molecular weight corresponds to that of RNase HII in a form of a monomer.

The RNase HII eluted as described above was used as an Afu RNase HII preparation.

An enzymatic activity was measured as described below using the thus obtained Afu RNase HII preparation. As a result, an RNase H activity was observed for the Afu RNase HII preparation.

(5) Measurement of activity of purified RNase H

(a) Preparation of reagent solutions used

Reaction mixture for determining activity: The following substances at the indicated final concentrations were contained in sterile water: 40 mM Tris-HCl (pH 7.7 at 37°C), 4 mM magnesium chloride, 1 mM DTT, 0.003% BSA, 4% glycerol and 24 µM poly(dT).

Poly[8-$^3$H]adenylic acid solution: 370 kBq of a poly[8-$^3$H]adenylic acid solution was dissolved in 200 µl of sterile water.

Polyadenylic acid solution: Polyadenylic acid was diluted to a concentration of 3 mM with sterile ultrapure water.

Enzyme dilution solution: The following substances at the indicated final concentrations were contained in sterile water: 25 mM Tris-HCl (pH 7.5 at 37°C), 5 mM 2-mercaptoethanol, 0.5 mM EDTA (pH 7.5 at 37°C), 30 mM sodium chloride and 50% glycerol.

Preparation of heat-denatured calf thymus DNA: 200 mg of calf thymus DNA was suspended and allowed to swell in 100 ml of TE buffer. The solution was diluted to a concentration of 1 mg/ml with sterile ultrapure water based on the absorbance measured at UV 260 nm. The diluted solution was heated at 100°C for 10 minutes and then rapidly cooled in an ice bath.

(b) Method for measuring activity

7 μl of the poly[8-$^3$H]adenylic acid solution was added to 985 μl of the reaction mixture for determining activity prepared in (a) above. The mixture was incubated at 37°C for 10 minutes. 8 μl of polyadenylic acid was added to the mixture to make the final concentration to 24 μM. The mixture was further incubated at 37°C for 5 minutes. Thus, 1000 μl of a poly[8-$^3$H]rA-poly-dT reaction mixture was prepared.

200 μl of the reaction mixture was then incubated at 30°C for 5 minutes. 1 μl of an appropriate serial dilution of an enzyme solution was added thereto. 50 μl each of samples was taken from the reaction mixture over time for use in subsequent measurement. The period of time in minutes from the addition of the enzyme to the sampling is defined as Y. 50 μl of a reaction mixture for total CPM or for blank was prepared by adding 1 μl of the enzyme dilution solution in place of an enzyme solution. 100 μl of 100 mM sodium pyrophosphate, 50 μl of the heat-denatured calf thymus DNA solution and 300 μl of 10% trichloroacetic acid (300 μl of ultrapure water for measuring total CPM) were added to the sample. The mixture was incubated at 0°C for 5 minutes, and then centrifuged at 10000 rpm for 10 minutes. After centrifugation, 250 μl of the resulting supernatant was placed in a vial. 10 ml of Aquasol-2 (NEN Life Science Products) was added thereto. CPM was measured in a liquid scintillation counter.

(c) Calculation of units

Unit value for each enzyme was calculated according to the following equation.

Unit/ml = {(measured CPM - blank CPM) x 1.2* x 20 x 1000 x dilution rate) x200 (μl) / (total CPM x Y (min.) x 50 (μl) x 9**)

1.2*: Amount in nmol of poly[8-$^3$H]rA-poly-dT contained in total CPM per 50 μl.

9**: Correction coefficient.

Unit value of a heat-resistant RNase H in the following Examples was calculated as follows.

1 mg of poly(rA) or poly(dT) (both from Amersham Pharmacia Biotech) was dissolved in 1 ml of 40 mM Tris-HCl (pH 7.7) containing 1 mM EDTA to prepare a poly(rA) solution and a poly(dT) solution.

The poly(rA) solution (to a final concentration of 20 μg/ml) and the poly(dT) solution (to a final concentration of 30 μg/ml) were then added to 40 mM Tris-HCl (pH 7.7) containing 4 mM·$MgCl_2$, 1 mM DTT, 0.003% BSA and 4% glycerol. The mixture was reacted at 37°C for 10 minutes and then cooled to 4°C to prepare a poly(rA)-poly(dT) solution. 1 μl of an appropriately diluted enzyme solution was added to 100 μl of the poly(rA)-poly(dT) solution. The mixture was reacted at 40°C for 10 minutes. 10 μl of 0.5 M EDTA was added thereto to terminate the reaction. Absorbance at 260 nm was then measured. As a control, 10 μl of 0.5 M EDTA was added to the reaction mixture, the resulting mixture was reacted at 40°C for 10 minutes, and the absorbance was then measured. A value (difference in absorbance) was obtained by subtracting the absorbance for the control from the absorbance for the reaction in the absence of EDTA. Thus, the concentration of nucleotide released from poly(rA)-poly(dT) hybrid by the enzymatic reaction was determined on the basis of the difference in absorbance. One unit of an RNase H was defined as an amount of enzyme that increases $A_{260}$ corresponding to release of 1 nmol of ribonucleotide in 10 minutes, which was calculated according to the following equation:

Unit = [Difference in Absorbance x Reaction

Volume (ml)] / 0.0152 x (110/100) x Dilution Rate

Example 1

(1) Preparation of genomic DNA

10 ml of blood was collected from each of seven human healthy donors after obtaining informed consent. A genomic DNA was prepared from 100 µl each of the blood using Dr. GenTLE (for blood) (Takara Bio). The concentrations of the thus obtained genomic DNA solutions were as follows:

Test sample no.1: 182 ng/µl
Test sample no.2: 150 ng/µl
Test sample no.3: 156 ng/µl
Test sample no.4: 204 ng/µl
Test sample no.5: 105 ng/µl
Test sample no.6: 172 ng/µl
Test sample no.7: 253 ng/µl

(2) Syntheses of primers and probes

Oligonucleotide primers for detection using ICAN reaction each having three RNA residues at the 3' terminus were designed and synthesized based on the nucleotide sequence of the human glutathione-S-transferase M1 (GSTM1) gene (GenBank accession no. X51451). Specifically, oligonucleotide primers GS-F (SEQ ID NO:6) and GS-R (SEQ ID NO:7) were synthesized. The oligonucleotide GS-F is a sense primer, and the oligonucleotide GS-R is an antisense primer. An oligonucleotide primer GS-R-bio (SEQ ID NO:8) in which GS-R is biotinylated at the 5' terminus was synthesized. A mixture of GS-R and GS-R-bio at a ratio of 9:1 (hereinafter referred to as GS-R-mix) was subjected to reaction. Furthermore, an oligonucleotide probe GS-D (SEQ ID NO:9) which is modified with FITC at the 5' terminus and used for detection of an ICAN reaction amplification product using ELISA was synthesized. GS-D is a sense probe. An oligonucleotide primer GS-Epc-F (SEQ ID NO:10) for preparing an ELISA positive control which serves as a standard for color development upon detection of an ICAN reaction amplification product using ELISA, and an oligonucleotide primer GS-Epc-R (SEQ ID NO:11) which is biotinylated at the 5' terminus were synthesized. The oligonucleotide GS-Epc-F is a sense primer, and the oligonucleotide GS-Epc-R is an antisense primer. The primers were designed such that the ELISA positive control becomes slightly shorter than the ICAN amplification product.

On the other hand, oligonucleotide primers for PCR were designed and synthesized. Specifically, oligonucleotide primers GS-PCR-F (SEQ ID NO:12) and GS-PCR-R (SEQ ID NO:13) were synthesized. The oligonucleotide GS-PCR-F is a sense primer, and the oligonucleotide GS-PCR-R is an antisense primer.

(3) Detection of GSTM1 gene using ICAN reaction

A reaction mixture of a total volume of 5 µl containing 50 pmol each of the synthetic oligonucleotide primers GS-F and GS-R-mix, 1 µl of a 0.05% propylenediamine aqueous solution and 1 µl of one of the test sample genomic DNA solutions prepared in (1) was heated at 98°C for two minutes and then at 58°C to anneal the primers to the template in Thermal Cycler Personal (Takara Bio). 20 µl of a mixture containing 0.625 mM dNTP mix, 40 mM Hepes-KOH buffer (pH 7.8), 125 mM potassium acetate, 5 mM magnesium acetate, 0.0125% bovine serum albumin, 1.25% dimethyl sulfoxide, 13.8 U of Afu RNase HII, 5.5 U of BcaBest DNA polymerase (Takara Bio) and sterile water was added to the heated mixture to make the final volume to 25 µl. The reaction mixture was incubated at 58°C for 1 hour. After reaction, 5 µl each of the reaction mixtures was subjected to electrophoresis on 3.0% agarose gel. As a result, amplification products derived from the GSTM1 gene were observed only when genomic DNAs derived from the test sample nos. 5 and 7 were used. Thus, it was confirmed that these test samples contain the GSTM1 gene. The results are shown in Figure 1. Figure 1 shows the pattern of agarose gel electrophoresis of the ICAN reaction products. Lanes 1, 2, 3, 4, 5, 6 and 7 represent the test sample nos. 1, 2, 3, 4, 5, 6 and 7, respectively.

On the other hand, the GSTM1 gene was detected by PCR using 1 µl each of the same test sample genomic DNA solutions. PCRs were carried out using primers GS-PCR-F (SEQ ID NO:12) and GS-PCR-R (SEQ ID NO:13), and the reaction mixtures were subjected to electrophoresis on 3.0% agarose gel. As a result, amplification products derived from the GSTM1 gene were observed only when genomic DNAs derived from the test sample nos. 5 and 7 were used. The results are shown in Figure 2. Figure 2 shows the pattern of agarose gel electrophoresis of the PCR reaction products. Lanes 1, 2, 3, 4, 5, 6 and 7 represent the test sample nos. 1, 2, 3, 4, 5, 6 and 7, respectively. As described above, the results obtained using the ICAN method were consistent with the results obtained using the PCR method.

(4) Detection of ICAN amplification product using ELISA method

1. Preparation of ELISA positive control

PCR was carried out using the primers GS-Epc-F and GS-Epc-R synthesized in Example 1-(2) above, and 200 ng of a genomic DNA from the promyelocytic cell line HL-60 as a template. The amplification product was appropriately diluted upon use in the ELISA as described below to result in absorbance of about 1. The concentration of the DNA was 340 amol/µl.

2. Detection using ELISA method

5 µl each of the respective reaction products obtained in Example 1-(3) above and 5 µl of the ELISA positive control solution were added to wells of an avidin plate (Labsystems) to which 50 µl of a hybridization buffer (5 x SSC, 1% Triton-X100, 1% BSA) had been dispensed. After reaction at room temperature for 15 minutes, the reaction mixtures were discarded, and each well was washed once with 360 µl of a washing buffer (25 mM Tris-HCl (pH7.5), 150 mM NaCl, 0.05% Tween 20). 50 µl of 0.02 N NaOH solution was added to each well. After reaction at room temperature for 3 minutes, 100 µl of the hybridization buffer containing 5 pmol of the GS-D probe was added thereto. After reaction at room temperature for 20 minutes, the reaction mixtures were discarded, and each well was washed twice with 360 µl of the washing buffer. 100 µl of the hybridization buffer containing a POD-labeled anti-FITC antibody was added to each well. After reaction at room temperature for 20 minutes, the reaction mixtures were discarded, and each well was washed four times with 360 µl of the washing buffer. 100 µl of a TMB solution (BioFX Laboratories) was added to each well. After reaction at room temperature for 10 minutes, 100 µl of 1 N sulfuric acid was added thereto in the order of the addition of the TMB solution. The absorbance at 450 nm was measured using a plate reader for each well. The value for the ELISA positive control was defined as the cut-off value. Test samples that resulted in higher absorbance values were determined to be positive for the GSTM1 gene.

As a result, the ICAN reaction products derived from the genomic DNAs from the test sample nos. 5 and 7 were determined to be positive for the GSTM1 gene. The results were similar to those obtained with determination using electrophoresis. The results are shown in Figure 3. Figure 3 is a graph representing the results of ELISA. The numbers on the horizontal axis 1, 2, 3, 4, 5, 6 and 7 represent the test sample nos. 1, 2, 3, 4, 5, 6 and 7. The number 8 represents the positive control. The longitudinal axis represents the absorbance at 450 nm.

Example 2

(1) Allele-specific detection of human CYP2C19(636)

A detection method for determining whether alleles are genetically homozygous or heterozygous (homo-type or hetero-type) at the 636th nucleotide in human CYP2C19 was examined.

Genomic DNA was prepared using Dr. GenTLE™ (Takara Bio) from 200 µl each of whole bloods obtained from healthy individuals (sample nos. 1-6) after obtaining informed consent. A reaction mixture of a total volume of 5 µl containing 160 ng of the prepared genomic DNA as a template, 50 pmol each of a synthetic oligonucleotide as a sense primer for specific detection of the allele of 636G (SEQ ID NO:14) or 636A (SEQ ID NO:15) and a synthetic oligonucleotide as an antisense primer (SEQ ID NO:16), and 1 µl of a 0.05% propylenediamine aqueous solution was heated at 98°C for two minutes and then at 53°C to anneal the primers to the template in Thermal Cycler Personal (Takara Bio). 20 µl of a mixture containing 0.625 mM dNTP mix, 40 mM Hepes-KOH buffer (pH 7.8), 125 mM potassium acetate, 5 mM magnesium acetate, 0.0125% bovine serum albumin, 1.25% dimethyl sulfoxide, 11 U of Afu RNase HII, 5.5 U of BcaBest DNA polymerase and sterile water was added to the heated mixture to make the final volume to 25 µl. The reaction mixture was incubated at 53°C for 1 hour. After reaction, 5 µl each of the reaction mixtures was subjected to electrophoresis on 3.0% agarose gel. The results are shown in Figures 4A-F. Figures 4A-F show the electrophoretic patterns that represent the results of typing using genomic DNAs extracted from the blood sample nos. 1-6 as templates. Lanes 1 and 2 represent the results obtained using the nucleotides of SEQ ID NO:14 (for detection of 636G) and SEQ ID NO:15 (for detection of 636A) as Nucleotides, respectively. Based on the patterns of amplification product shown in Figures 4A-F, the alleles of the respective blood samples at the 636th nucleotide in CYP2C19 were typed as follows: 1: G/A, 2: G/G, 3: G/A, 4: G/G, 5: G/G, and 6: G/G.

On the other hand, PCRs were carried out using the same genomic DNAs as templates and primers represented by SEQ ID NOS:17 and 18. The resulting PCR amplification products were treated with BamHI and the reaction mixtures were subjected to electrophoresis on 3.0% agarose gel for typing by the PCR-RFLP method. The results are shown in Figure 4G. Figure 4G shows an electrophoretic pattern that represents the results of typing by the PCR-RFLP method using genomic DNAs prepared from the blood sample nos. 1-6 as templates. Lanes 1-6 represent the results obtained using the genomic DNAs extracted from the blood sample nos. 1-6 as templates, respectively. Based on the results of electrophoresis shown in Figure 4G (i.e., the digestion pattern of PCR amplification products obtained using DNAs prepared from the respective blood samples as templates), the alleles at the 636th nucleotide in CYP2C19 were typed as follows: 1: G/A, 2: G/G, 3: G/A, 4: G/G, 5: G/G, and 6: G/G. The results were consistent with the above-mentioned ones.

(2) Allele-specific detection of human CYP2C19(636) using ELISA

An SNP typing reaction was carried out according to the method as described in Example 2-(1) using 160 ng of genomic DNA prepared from HL-60 (G/G), or 150 ng of genomic DNA prepared from the blood sample no. 1 (G/A) or 230 ng of genomic DNA prepared from the blood sample no. 4 (G/G) as described in Example 2-(1) as a template, a Nucleotide as a primer for specific detection of the allele of 636G (SEQ ID NO:14) or 636A (SEQ ID NO:15), and a primer (1% of the primer was labeled at the 5' terminus with biotin) as an antisense primer (SEO ID NO:19). 2.5 µl each of the typing reaction products was added to wells of an avidin plate (Labsystems) to which 50 µl of a hybridization buffer (5 x SSC, 1% Triton-X100, 1% BSA) had been dispensed. After reaction at room temperature for 15 minutes, the reaction mixtures were discarded, and each well was washed once with 360 µl of a washing buffer (25 mM Tris-HCl (pH7.5), 150 mM NaCl, 0.05% Tween 20). 50 µl of 0.017 N NaOH solution was added to each well. After reaction at room temperature for 3 minutes, 100 µl of the hybridization buffer containing 2.5 pmol of a DNA probe (SEQ ID NO: 20) labeled at the 5' terminus with FITC was added thereto. After reaction at room temperature for 20 minutes, the reaction mixtures were discarded, and each well was washed twice with 360 µl of the washing buffer. 100 µl of the hybridization buffer containing a POD-labeled anti-FITC antibody was added to each well. After reaction at room temperature for 20 minutes, the reaction mixtures were discarded, and each well was washed four times with 360 µl of the washing buffer. 100 µl of a TMB solution (BioFX Laboratories) was added to each well. After reaction at room temperature for 10 minutes, 100 µl of 1 N sulfuric acid was added thereto in the order of the addition of the TMB solution. The absorbance at 450 nm was measured using a plate reader for each well. The results are shown in Figure 5. In Figure 5, ■ and □ represent the results of typing reactions using Nucleotides represented by SEQ ID NOS:14 and 15 for specific detection of the alleles of 636G and 636A, respectively. The numbers on the horizontal axis 1, 2 and 3 represent the results of typing of HL-60, the blood sample no. 1 and the blood sample no. 4, respectively. The number 4 represents the positive control. As seen from Figure 5, when the value for'the ELISA positive control was defined as the cut-off value, the alleles of the respective samples at the 636th nucleotide in CYP2C19 were typed, providing results consistent with the alleles of these genomic DNAs.

Example 3

Typing of SmaI polymorphism (T6235C)) in human CYP1A1 3'-flanking region was carried out.

Nucleotides represented by SEQ ID NOS:21 and 22 as primers for specific detection of human CYP1A1 alleles having C and T at the 6235th nucleotide were synthesized. The following reaction was carried out using the Nucleotide as an antisense primer and a primer represented by SEQ ID NO:23 (1% of the primer was biotinylated at the 5' terminus) as a sense primer. A reaction mixture of a total volume of 5 µl containing 50 pmol each of the synthetic oligonucleotide primers (sense and antisense primers), 1 µl of a 0.05% propylenediamine aqueous solution and 100 ng of one of genomic DNAs for which the CYP1A1 alleles at the 6325th nucleotide had been confirmed to be (C/C) and (T/T) by PCR-RFLP was heated at 98°C for two minutes and then at 53°C to anneal the primers to the template in Thermal Cycler Personal (Takara Bio). 20 µl of a mixture containing 0.625 mM dNTP mix, 40 mM Hepes-KOH buffer (pH 7.8), 125 mM potassium acetate, 5 mM magnesium acetate, 0.0125% bovine serum albumin, 1.25% dimethyl sulfoxide, 14 U of Afu RNase HII, 5.5 U of BcaBest DNA polymerase (Takara Bio) and sterile water was added to the heated mixture to make the final volume to 25 µl. The reaction mixture was incubated at 60°C for 1 hour. After reaction, 5 µl each of the reaction mixtures was subjected to electrophoresis on 3.0% agarose gel. The results are shown in Figure 6. In the electrophoretic pattern shown in Figure 6, lanes 1, 2, 5 and 6 represent the results obtained using the Nucleotide represented by SEQ ID NO:21, and lanes 3, 4, 7 and 8 represent the results obtained using the Nucleotide represented by SEQ ID NO:22. Lanes 1-4 represent the results obtained using the genomic DNA for which the CYP1A1 allele at the 6235th nucleotide had been confirmed to be (C/C) as a template, and lanes 5-8 represent the results obtained using the genomic DNA for which the CYP1A1 allele at the 6235th nucleotide had been confirmed to be (T/T) as a template. Based on these results, it was confirmed that allele-specific detection can be carried out using the Nucleotides.

Detection of the reaction products using ELISA was carried out. 5 µl each of the reaction products was added to wells of an avidin plate (Labsystems) to which 50 µl of a hybridization buffer (5 x SSC, 1% Triton-X100, 1% BSA) had been dispensed. After reaction at room temperature for 15 minutes, the reaction mixtures were discarded, and each well was washed once with 360 µl of a washing buffer (25 mM Tris-HCl (pH7.5), 150 mM NaCl, 0.05% Tween 20). 50 µl of 0.017 N NaOH solution was added to each well. After reaction at room temperature for 3 minutes, 100 µl of the hybridization buffer containing 5 pmol of a DNA probe (SEQ ID NO:24) labeled at the 5' terminus with FITC was added thereto. After reaction at room temperature for 20 minutes, the reaction mixtures were discarded, and each well was washed twice with 360 µl of the washing buffer. 100 µl of the hybridization buffer containing a POD-labeled anti-FITC antibody was added to each well. After reaction at room temperature for 20 minutes, the reaction mixtures were discarded, and each well was washed four times with 360 µl of the washing buffer. 100 µl of a TMB solution (BioFX Laboratories) was added to each well. After reaction at room temperature for 10 minutes, 100 µl of 1 N sulfuric acid was added thereto in the order of the addition of the TMB solution. The absorbance at 450 nm was measured using a plate reader for each well. The results are shown in Figure 7. Among the results of ELISA shown in Figure 7, lanes 1, 2, 5 and 6 represent the results obtained using the Nucleotide represented by SEQ ID NO:21, and lanes 3, 4, 7 and 8 represent the results obtained using the Nucleotide represented by SEQ ID NO:22. Lanes 1-4 represent the results obtained using the genomic DNA for which the CYP1A1 allele at the 6235th nucleotide had been confirmed to be (C/C) as a template, and lanes 5-8 represent the results obtained using the genomic DNA for which the CYP1A1 allele at the 6235th nucleotide had been confirmed to be (T/T) as a template. As seen from Figure 7, when the value 1 of absorbance at 450 nm was defined as the cut-off value, typing could be carried out, providing results consistent with the alleles of these genomic DNAs.

Example 4

Typing of a single nucleotide polymorphism in human ALDH2 (aldehyde dehydrogenase 2) (exon 12, 487Glu→Lys) was carried out.

Nucleotides having the nucleotide sequences of SEQ ID NOS:25 and 26 as primers for specific detection of single nucleotide polymorphic alleles having G and A in exon 12 of human ALDH2 were synthesized. The following reaction was carried out using one of the Nucleotides as an antisense primer and a primer (0.5% of the primer was biotinylated at the 5' terminus) as a sense primer (SEQ ID NO:27). A reaction mixture of a total volume of 5 µl containing 50 pmol each of the synthetic oligonucleotide primers (sense and antisense primers), 1 µl of a 0.05% propylenediamine aqueous solution and 100 ng of one of genomic DNAs for which the single nucleotide polymorphic alleles in exon 12 of ALDH2 had been confirmed to be (G/G), (A/A) and (G/A) was heated at 98°C for two minutes and then at 54°C to anneal the primers to the template in Thermal Cycler Personal (Takara Bio). 20 µl of a mixture containing 0.625 mM dNTP mix, 40 mM Hepes-KOH buffer (pH 7.8), 125 mM potassium acetate, 5 mM magnesium acetate, 0.0125% bovine serum albumin, 1.25% dimethyl sulfoxide, 14 U of Afu RNase HII, 5.5 U of BcaBest DNA polymerase (Takara Bio) and sterile water was added to the heated mixture to make the final volume to 25 µl. The reaction mixture was incubated at 54°C for 1 hour. The resulting reaction products were subjected to detection using ELISA. 5 µl each of the reaction mixtures was added to wells of an avidin plate (Labsystems) to which 50 µl of a hybridization buffer (5 x SSC, 1% Triton-X100, 1% BSA) had been dispensed. After reaction at room temperature for 15 minutes, the reaction mixtures were discarded, and each well was washed once with 360 µl of a washing buffer (25 mM Tris-HCl (pH7.5), 150 mM NaCl, 0.05% Tween 20). 50 µl of 0.02 N NaOH solution was added to each well. After reaction at room temperature for 3 minutes, 100 µl of the hybridization buffer containing 5 pmol of a DNA probe (SEQ ID NO:28) labeled at the 5' terminus with FITC was added thereto. After reaction at room temperature for 20 minutes, the reaction mixtures were discarded, and each well was washed twice with 360 µl of the washing buffer. 100 µl of the hybridization buffer containing a POD-labeled anti-FITC antibody was added to each well. After reaction at room temperature for 20 minutes, the reaction mixtures were discarded, and each well was washed four times with 360 µl of the washing buffer. 100 µl of a TMB solution (BioFX Laboratories) was added to each well. After reaction at room temperature for 10 minutes, 100 µl of 1 N sulfuric acid was added thereto in the order of the addition of the TMB solution. The absorbance at 450 nm was measured using a plate reader for each well. The results are shown in Figure 8. Figure 8 is a graph representing the results of ELISA. The longitudinal axis represents the absorbance upon ELISA. The horizontal axis represents the sample numbers. The numbers 1, 3 and 5 on the horizontal axis represent the results obtained using the Nucleotide having a nucleotide sequence of SEQ ID NO:25 for detecting one having G for the single nucleotide polymorphism in exon 12 of ALDH2 (for detecting the active type), and the numbers 2, 4 and 6 represent the results obtained using the Nucleotide having a.nucleotide sequence of SEQ ID NO:26 for detecting one having A for the single nucleotide polymorphism in exon 12 of ALDH2 (for detecting the inactive type). Lanes 1 and 2 represent the results obtained using the genomic DNA for which the single nucleotide polymorphism in exon 12 of ALDH2 had been confirmed to be (G/G) as a template, lanes 3 and 4 represent the results obtained using the genomic DNA for which the single.nucleotide polymorphism in exon 12 of ALDH2 had been confirmed to be (G/A) as a template, and lanes 5 and 6 represent the results obtained using the genomic DNA for which the single nucleotide polymorphism in exon 12 of ALDH2 had been confirmed to be (A/A) as a template. As seen from Figure 8, when the value 1 of absorbance at 450 nm was defined as the cut-off value, typing could be carried out, providing results consistent with the alleles of these genomic DNAs. Thus, it was confirmed that the polymorphism of human aldehyde dehydrogenase 2 can be accurately detected using the method of the present invention.

Example 5

(1) Syntheses of primers and probes

Oligonucleotide primers for detection using ICAN reaction each having three RNA residues at the 3' terminus were designed and synthesized based on the nucleotide sequence of the human glutathione-S-transferase T1 (GSTT1) gene (GenBank accession no. AB057594). Specifically, oligonucleotide primers GST1-F (SEQ ID NO:29) and GST1-R (SEQ ID NO:30) were synthesized. The oligonucleotide GST1-F is a sense primer, and the oligonucleotide GST1-R is an antisense primer. An oligonucleotide primer GST1-F-bio (SEQ ID NO:31) in which GST1-F is biotinylated at the 5' terminus was synthesized. A mixture of GST1-F and GST1-F-bio at a ratio of 49:1 (hereinafter referred to as GST1-F-mix) was subjected to reaction. Furthermore, an oligonucleotide probe GST1-D (SEQ ID NO:32) which is modified with FITC at the 5' terminus and used for detection of an ICAN reaction amplification product using ELISA was synthesized. GST1-D is an antisense probe.

On the other hand, oligonucleotide primers for PCR were designed and synthesized. Specifically, oligonucleotide primers GST1-PCR-F (SEQ ID NO:33) and GST1-PCR-R (SEQ ID NO:34) were synthesized. The oligonucleotide GST1-PCR-F is a sense primer, and the oligonucleotide GST1-PCR-R is an antisense primer.

(2) Detection of GSTT1 gene using ICAN reaction

A reaction mixture of a total volume of 5 µl containing 50 pmol each of the synthetic oligonucleotide primers GST1-F-mix and GST1-R, 1 µl of a 0.05% propylenediamine aqueous solution and 1 µl of one of the test sample genomic DNA solutions prepared in Example 1-(1) was heated at 98°C for two minutes and then at 58°C to anneal the primers to the template in Thermal Cycler Personal (Takara Bio). 20 µl of a mixture containing 0.625 mM dNTP mix, 40 mM Hepes-KOH buffer (pH 7.8), 125 mM potassium acetate, 5 mM magnesium acetate, 0.0125% bovine serum albumin, 1.25% dimethyl sulfoxide, 13.8 U of Afu RNase HII (Takara Bio), 5.5 U of BcaBest DNA polymerase (Takara Bio) and sterile water was added to the heated mixture to make the final volume to 25 µl. The reaction mixture was incubated at 58°C for 1 hour. After reaction, 5 µl each of the reaction mixtures was subjected to electrophoresis on 3.0% agarose gel. As a result, amplification products derived from the GSTT1 gene were observed when the genomic DNAs derived from the test sample nos. 1, 2, 4, 5 and 6 were used. Thus, it was confirmed that these test samples contain the GSTT1 gene. The results are shown in Figure 9. Figure 9 shows the pattern of agarose gel electrophoresis of the ICAN reaction products. Lanes 1, 2, 3, 4, 5, 6 and 7 represent the test sample nos. 1, 2, 3, 4, 5, 6 and 7, respectively.

On the other hand, the GSTT1 gene was detected by PCR using 1 µl each of the same test sample genomic DNA solutions. PCRs were carried out using primers GST1-PCR-F and GST1-PCR-R, and the reaction mixtures were subjected to electrophoresis on 3.0% agarose gel. As a result, amplification products derived from the GSTT1 gene were observed when genomic DNAs derived from the test sample nos. 1, 2, 4, 5 and 6 were used. The results are shown in Figure 10. Figure 10 shows the pattern of agarose gel electrophoresis of the PCR reaction products. Lanes 1, 2, 3, 4, 5, 6 and 7 represent the test sample nos. 1, 2, 3, 4, 5, 6 and 7, respectively. As described above, the results obtained using the ICAN method were consistent with the results obtained using the PCR method.

(3) Detection of ICAN amplification product using ELISA method

5 µl each of the respective reaction products obtained in Example 5-(2) above was added to wells of an avidin plate (Labsystems) to which 50 µl of a hybridization buffer (5 x SSC, 1% Triton-X100, 1% BSA) had been dispensed. After reaction at room temperature for 15 minutes, the reaction mixtures were discarded, and each well was washed once with 360 µl of a washing buffer (25 mM Tris-HCl (pH7.5), 150 mM NaCl, 0.05% Tween 20). 50 µl of 0.02 N NaOH solution was added to each well. After reaction at room temperature for 3 minutes, 100 µl of the hybridization buffer containing 5 pmol of the GST1-D probe was added thereto. After reaction at room temperature for 15 minutes, the reaction mixtures were discarded, and each well was washed twice with 360 µl of the washing buffer. 100 µl of the hybridization buffer containing a POD-labeled anti-FITC antibody was added to each well. After reaction at room temperature for 20 minutes, the reaction mixtures were discarded, and each well was washed four times with 360 µl of the washing buffer. 100 µl of a TMB solution (BioFX Laboratories) was added to each well. After reaction at room temperature for 10 minutes, 100 µl of 1 N sulfuric acid was added thereto in the order of the addition of the TMB solution. The absorbance at 450 nm was measured using a plate reader for each well. Test samples that resulted in absorbance of 2 or more were determined to be positive for the GSTT1 gene.

As a result, the ICAN reaction products derived from the genomic DNAs from the test sample nos. 1, 2, 4, 5 and 6 were determined to be positive for the GSTT1 gene. The results were similar to those obtained with determination using electrophoresis. The results are shown in Figure 11. Figure 11 is a graph representing the results of ELISA. The horizontal axis represents the test sample nos. The longitudinal axis represents the absorbance at 450 nm.

Example 6

(1) Syntheses of primers and probes

An internal positive control used upon typing of a human gene according to the present invention was examined. ICAN primers for the internal control were constructed as follows.

Oligonucleotide primers for ICAN reaction each having three RNA residues at the 3' terminus were designed and synthesized based on the nucleotide sequence of the human β-globin gene (GenBank accession no. AF007546). Specifically, oligonucleotide primers βG-F1 (SEQ ID NO:35), βG-R1 (SEQ ID NO:36), βG-F2 (SEQ ID NO:37), βG-R2 (SEQ ID NO:38) and βG-R3 (SEQ ID NO:39) were synthesized. The oligonucleotide primers βG-F1 and βG-F2 are sense primers, and the oligonucleotide primers βG-R1, βG-R2 and βG-R3 are antisense primers. Oligonucleotide primers βG-F1-Bio (SEQ ID NO:40), βG-R2-Bio (SEQ ID NO:41) and βG-R3-Bio (SEQ ID NO:42) in which βG-F1, βG-R2 and βG-R3 are biotinylated at the 5' termini were synthesized. Mixtures of βG-F1 and βG-F1-Bio, βG-R2 and βG-R2-Bio, and βG-R3 and βG-R3-Bio at ratios of 49:1 (hereinafter referred to as βG-F1-mix, βG-R2-mix and βG-R3-mix, respectively) were subjected to reactions. Furthermore, oligonucleotide probes βG-D1 (SEQ ID NO:43) and βG-D2 (SEQ ID NO:44) which are modified with FITC at the 5' termini and used for detection of an ICAN reaction product using ELISA were synthesized.

(2) Detection of β-globin using ICAN

The following reaction was carried out using a combination of βG-F1-mix and βG-R1 (primer set 1), βG-F2 and βG-R2-mix (primer set 2), or βG-F2 and βG-R3-mix (primer set 3) as a combination of primers for an ICAN reaction. A reaction mixture of a total volume of 5 μl containing 50 pmol each of the synthetic oligonucleotide primers, 1 μl of a 0.05% propylenediamine aqueous solution and 100 ng, 10 ng, 1 ng or 0 ng of HL-60 genomic DNA was heated at 98°C for two minutes and then at 56°C to anneal the primers to the template in Thermal Cycler Personal (Takara Bio). 20 μl of a mixture containing 0.625 mM dNTP mix, 40 mM Hepes-KOH buffer (pH 7.8), 125 mM potassium acetate, 5 mM magnesium acetate, 0.0125% bovine serum albumin, 1.25% dimethyl sulfoxide, 13.8 U of Afu RNase HII, 5.5 U of BcaBest DNA polymerase (Takara Bio) and sterile water was added to the heated mixture to make the final volume to 25 μl. The reaction mixture was incubated at 56°C for 1 hour.

After reaction, 5 μl each of the reaction products was added to wells of an avidin plate (Labsystems) to which 50 μl of a hybridization buffer (5 x SSC, 1% Triton-X100, 1% BSA) had been dispensed. After reaction at room temperature for 15 minutes, the reaction mixtures were discarded, and each well was washed once with 360 μl of a washing buffer (25 mM Tris-HCl (pH7.5), 150 mM NaCl, 0.05% Tween 20). 50 μl of 0.02 N NaOH solution was added to each well. After reaction at room temperature for 3 minutes, 100 μl of the hybridization buffer containing 5 pmol of βG-D1 (in case of the primer set 1) or βG-D2 (in case of the primer set 2 or 3) was added thereto. After reaction at room temperature for 20 minutes, the reaction mixtures were discarded, and each well was washed twice with 360 μl of the washing buffer. 100 μl of the hybridization buffer containing a POD-labeled anti-FITC antibody was added to each well. After reaction at room temperature for 20 minutes, the reaction mixtures were discarded, and each well was washed four times with 360 μl of the washing buffer.

After washing, 100 μl of a TMB solution (BioFX Laboratories) was added to each well. After reaction at room temperature for 10 minutes, 100 μl of 1 N sulfuric acid was added thereto in the order of the addition of the TMB solution. The absorbance at 450 nm was measured using a plate reader for each well. The results are shown in Figures 12-14. Figures 12, 13 and 14 represent the results of ELISA using the primer sets 1, 2 and 3, respectively. The horizontal axes represent the concentration of HL-60 genomic DNA. The longitudinal axes represent absorbance at 450 nm.

As shown in Figures 12-14, the β-globin gene could be detected from the HL-60 genomic DNA using either of the primer sets 1, 2 and 3.

(3) Multiplex ICAN

Genetic typing reactions for GSTM1 and GSTT1 by multiplex ICAN reactions using the β-globin gene examined in (2) above as an internal positive control were conducted. The reactions were carried out as follows.

Specifically, GS-F and GS-R-mix as described in Example 1 as oligonucleotide primers for GSTM1 typing reaction, and GST1-F-mix and GST1-R as described in Example 5 as oligonucleotide primers for GSTT1 typing reaction were used. A reaction mixture of a total volume of 5 μl containing 25 pmol each of the primers for typing and the primer set 1 or 2 for detecting the β-globin gene, 1 μl of a 0.05% propylenediamine aqueous solution and 100 ng of genomic DNA from a test sample (test sample no. 1: without deletion of both GSTM1 and GSTT1; test sample no. 2: with deletion of both GSTM1 and GSTT1) was heated at 98°C for two minutes and then at 56°C to anneal the primers to the template in Thermal Cycler Personal (Takara Bio). 20 μl of a mixture containing 0.625 mM dNTP mix, 40 mM Hepes-KOH buffer (pH 7.8), 125 mM potassium acetate, 5 mM magnesium acetate, 0.0125% bovine serum albumin, 1.25% dimethyl sulfoxide, 13.8 U of Afu RNase HII, 5.5 U of BcaBest DNA polymerase (Takara Bio) and sterile water was added to the heated mixture to make the final volume to 25 μl. The reaction mixture was incubated at 56°C for 1 hour.

After reaction, 5 μl each of the reaction products was added to wells of an avidin plate (Labsystems) to which 50 μl of a hybridization buffer (5 x SSC, 1% Triton-X100, 1% BSA).had been dispensed. After reaction at room temperature for 15 minutes, the reaction mixtures were discarded, and each well was washed once with 360 μl of a washing buffer (25 mM Tris-HCl (pH7.5), 150 mM NaCl, 0.05% Tween 20). 50 μl of 0.02 N NaOH solution was added to each well. After reaction at room temperature for 3 minutes, 100 μl of the hybridization buffer containing 5 pmol of GS-D (in case of detection of GSTM1), GST1-D (in case of detection of GSTT1), βG-D1 (in case of the primer set 1) or βG-D2 (in case of the primer set 2) was added thereto. After reaction at room temperature for 20 minutes, the reaction mixtures were discarded, and each well was washed twice with 360 μl of the washing buffer.

After washing, 100 μl of the hybridization buffer containing a POD-labeled anti-FITC antibody was added to each well. After reaction at room temperature for 20 minutes, the reaction mixtures were discarded, and each well was washed four times with 360 μl of the washing buffer. 100 μl of a TMB solution (BioFX Laboratories) was added to each well. After reaction at room temperature for 10 minutes, 100 μl of 1 N sulfuric acid was added thereto in the order of the addition of the TMB solution. The absorbance at 450 nm was measured using a plate reader for each well.

The results are shown in Figures 15, 16, 17 and 18. Figures 15 and 16 represent the results of GSTM1 typing using the primer sets 1 and 2 for detecting the β-globin gene, respectively. Figures 17 and 18 represent the results of GSTT1 typing using the primer sets 1 and 2 for detecting the β-globin gene, respectively. In the figures, the horizontal axes represent the test sample numbers. The longitudinal axes represent absorbance at 450 nm. In Figures 15 and 16, the dark bars represent the results for GSTM1 typing and the shaded bars represent the results for detection of the β-globin gene. Similarly, in Figures 17 and 18, the dark bars represent the results for GSTT1 typing and the shaded bars represent the results for detection of the β-globin gene.

As shown in Figures 15 to 18, it was confirmed that genetic typing reactions for GSTM1 and GSTT1 by multiplex ICAN reactions using the β-globin gene as an internal positive control are possible.

Example 7

A kit for use in the method of the present invention was constructed. A method for typing a human genetic polymorphism by ICAN and/or UCAN using alkali denaturation in combination was examined.

(1) Construction of a kit for typing of a polymorphism in the ALDH2 gene and typing using the kit

A reaction for typing of a single nucleotide polymorphism in ALDH2 was carried out as follows. First, the following reagents (for 16 reactions) were prepared.

Reagent 1: 0.1 M Hepes solution (80 μl) containing 50 μM each of a Nucleotide having a nucleotide sequence of SEQ ID NO:25, and a chimeric oligonucleotide primer having a nucleotide sequence of SEQ ID NO:27 (0.5% of the primer was biotinylated at the 5' terminus);
Reagent 2: 0.1 M Hepes solution (80 μl) containing 50 μM each of a Nucleotide having a nucleotide sequence of SEQ ID NO:26, and a chimeric oligonucleotide primer having a nucleotide sequence of SEQ ID NO:27 (0.5% of the primer was biotinylated at the 5' terminus);
Reagent 3: a solution (80 μl) containing 32 mM Hepes-KOH buffer (pH 7.8), 100 mM KOAc, 4 mM $Mg(OAc)_2$, 2.5 mM dNTP mix, 0.05% bovine serum albumin, 2.8 U/μl of Afu RNase HII and 1.1 U/μl of BcaBest DNA polymerase (Takara Bio); and
Reagent 4: a solution (160 μl) containing 14 mM Hepes-KOH buffer (pH 7.8), 200 mM KOAc, 8 mM $Mg(OAc)_2$ and 2.5% dimethyl sulfoxide.

The following procedure was conducted using the above-mentioned reagents. Specifically, an equal volume of 0.1 N NaOH was added to a solution containing genomic DNA for which the single nucleotide polymorphic allele in exon 12 of ALDH2 had been confirmed to be (G/G), (G/A) or (A/A) at a concentration of 40 ng/μl. The mixture was

allowed to stand at room temperature for 5 minutes to obtain a sample solution. 5 μl of the sample solution was added to 5 μl of the Reagent 1 or 2. 15 μl of a reaction mixture obtained by mixing 5 μl of the Reagent 3 and 10 μl of the Reagent 4 was further added thereto. The mixture was incubated at 54°C for 1 hour. The resulting reaction products were detected according to the method as described in Example 4. The results are shown in Figure 19.

Figure 19 is a graph representing the results of ELISA detection of a genetic polymorphism in human ALDH2 using the kit of the present invention. The horizontal axis represents the sample numbers. The longitudinal axis represents the absorbance at 450 nm. In the figures, lanes 1, 3 and 5 represent the results obtained using the Nucleotide represented by SEQ ID NO:25, and lanes 2, 4 and 6 represent the results obtained using the Nucleotide represented by SEQ ID NO:26. Lanes 1 and 2 represent the results obtained using the genomic DNA for which the single nucleotide polymorphism in exon 12 of the ALDH2 gene had been confirmed to be (G/G) as a template; lanes 3 and 4 represent the results obtained using the genomic DNA for which the single nucleotide polymorphism in exon 12 of the ALDH2 gene had been confirmed to be (G/A) as a template; and lanes 5 and 6 represent the results obtained using the genomic DNA for which the single nucleotide polymorphism in exon 12 of the ALDH2 gene had been confirmed to be (A/A) as a template. As shown in Figure 19, when the value 1 of absorbance at 450 nm was defined as the cut-off value, typing could be carried out, providing results consistent with the alleles of these genomic DNAs. Thus, it was confirmed that the kit of the present invention can be used for a method for typing a genetic polymorphism. In addition, it was confirmed that alkali denaturation may be used in combination in the method of the present invention.

(2) Construction of a kit for typing of a deletion polymorphism in GSTM1 and typing using the kit

A reaction for typing of a deletion polymorphism in GSTM1 was carried out as follows. First, the following reagents (for 32 reactions) were prepared.

Reagent 1: 0.1 M Hepes solution (160 μl) containing 25 μM each of the oligonucleotide primers GS-F and GS-R-mix as described in Example 1, as well as 25 μM each of the oligonucleotide primers in the primer set 1 for detecting the β-globin gene as described in Example 6-(2);

Reagent 2: a solution (160 μl) containing 32 mM Hepes-KOH buffer (pH 7.8), 100 mM KOAc, 4 mM $Mg(OAc)_2$, 2.5 mM dNTP mix, 0.05% bovine serum albumin, 2.8 U/μl of Afu RNase HII and 1.1 U/μl of BcaBest DNA polymerase (Takara Bio); and

Reagent 3: a solution (320 μl) containing 14 mM Hepes-KOH buffer (pH 7.8), 200 mM KOAc, 8 mM $Mg(OAc)_2$ and 2.5% dimethyl sulfoxide.

The following procedure was conducted using the above-mentioned reagents. Specifically, an equal volume of 0.1 N NaOH was added to a solution containing genomic DNA from a test sample (test sample no. 1: without GSTM1 deletion; test sample no. 2: with GSTM1 deletion) at a concentration of 40 ng/μl. The mixture was allowed to stand at room temperature for 5 minutes to obtain a sample solution. 5 μl of the sample solution was added to 5 μl of the Reagent 1. 15 μl of a reaction mixture obtained by mixing 5 μl of the Reagent 2 and 10 μl of the Reagent 3 was added thereto. The mixture was incubated at 56°C for 1 hour. The resulting reaction products were detected according to the method as described in Example 6-(3). The results are shown in Figure 20.

Figure 20 is a graph representing the results of ELISA detection of a genetic polymorphism in human GSTM1 using the kit of the present invention. The horizontal axis represents the sample numbers. The longitudinal axis represents the absorbance at 450 nm. In the figure, the dark bars represent the results for detection of GSTM1 and the shaded bars represent the results for detection of the β-globin. As shown in Figure 20, it was possible to conduct genetic typing of GSTM1 by multiplex ICAN reaction using the β-globin gene as an internal positive control under the above-mentioned conditions. Thus, it was confirmed that the kit of the present invention can be used for a method for typing a genetic polymorphism. In addition, it was confirmed that alkali denaturation can be used in combination even if a distinct subject to be detected is used.

(3) Construction of a kit for typing of a deletion polymorphism in GSTT1 and typing using the kit

Detection of GSTT1 was examined in a manner similar to (2) above. Specifically, Reagents having the same compositions as those described in (2) above were prepared except that the oligonucleotide primers GST1-F-mix and GST1-R as described in Example 5 were used. Typing could be carried out when typing was carried out using the Reagents according to the method as described above for GSTM1. Thus, it was confirmed that the kit of the present invention can be used for a method for typing a genetic polymorphism. In addition, it was confirmed that alkali denaturation can be used in combination in any case.

Industrial Applicability

The Nucleotide of the present invention and the method for detecting a base substitution, an insertion mutation or a deletion mutation using the Nucleotide as described above are useful for detection of a naturally occurring or artificially introduced base substitution, insertion mutation or deletion mutation.

According to the present invention, the presence of a base substitution, an insertion mutation or a deletion mutation in a target nucleic acid can be conveniently detected with reproducibility. The method of the present invention can be readily combined with a known nucleic acid amplification method, and can be used to detect a base substitution, an insertion mutation or a deletion mutation with high sensitivity. By further using a Nucleotide having a suitable sequence in combination, it is possible to obtain information about the presence of a base substitution, an insertion mutation or a deletion mutation and the type of the substitution, insertion or deletion.

The present invention can be used for detecting or identifying a base substitution, an insertion mutation or a deletion mutation generated in a genomic DNA in an organism such as a polymorphism or a variation (e.g., an SNP), and the present invention is useful in fields of genomic drug discovery or genomic therapy as well (e.g., screening of disease genes or analyses of drug sensitivities in humans).

Sequence Listing Free Text

SEQ ID NO:2: PCR primer AfuNde for cloning a gene encoding a polypeptide having a RNaseHII activity from Archaeoglobus fulgidus

SEQ ID NO:3: PCR primer AfuBam for cloning a gene encoding a polypeptide having a RNaseHII activity from Archaeoglobus fulgidus

SEQ ID NO:6: Chimeric oligonucleotide primer to amplify a portion of human GSTM1 gene. "nucleotides 20 to 22 are ribonucleotides-other nucleotides are deoxyribonucleotides"

SEQ ID NO:7: Chimeric oligonucleotide primer to amplify a portion of human GSTM1 gene. "nucleotides 21 to 23 are ribonucleotides-other nucleotides are deoxyribonucleotides"

SEQ ID NO:8: Chimeric oligonucleotide primer to amplify a portion of human GSTM1 gene. "nucleotides 21 to 23 are ribonucleotides-other nucleotides are deoxyribonucleotides, and 5' end is labeled by biotin"

SEQ ID NO:9: Oligonucleotide probe for detecting a portion of human GSTM1 gene. "5' end is labeled by FITC"

SEQ ID NO:10: Designed PCR primer to amplify a portion of human GSTM1 gene

SEQ ID NO:11: Designed PCR primer to amplify a portion of human GSTM1 gene

SEQ ID NO:12: Designed PCR primer to amplify a portion of human GSTM1 gene

SEQ ID NO:13: Designed PCR primer to amplify a portion of human GSTM1 gene

SEQ ID NO:14: Chimeric oligonucleotide to detect the nucleotide substitution on human CYP2C19 gene. "nucleotides 13 to 15 are ribonucleotides-other nucleotides are deoxyribonucleotides and the 3'-OH group of the nucleotide at 3'end is protected with amino hexyl group"

SEQ ID NO:15: Chimeric oligonucleotide to detect the nucleotide substitution on human CYP2C19 gene. "nucleotides 13 to 15 are ribonucleotides-other nucleotides are deoxyribonucleotides and the 3'-OH group of the nucleotide at 3'end is protected with amino hexyl group

SEQ ID NO:16: Chimeric oligonucleotide primer to amplify a portion of human CYP2C19 gene. "nucleotides 19 to 21 are ribonucleotides-other nucleotides are deoxyribonucleotides"

SEQ ID NO:17: Designed PCR primer to amplify a portion of human CYP2C19 gene

SEQ ID NO:18: Designed PCR primer to amplify a portion of human CYP2C19 gene

SEQ ID NO:19: Chimeric oligonucleotide primer to amplify a portion of human CYP2C19 gene. "nucleotides 19 to 21 are ribonucleotides-other nucleotides are deoxyribonucleotides, and 5' end is labeled by biotin"

SEQ ID NO:20: Oligonucleotide probe for detecting a portion of human CYP2C19 gene. "5' end is labeled by FITC"

SEQ ID NO:21: Chimeric oligonucleotide to detect the nucleotide substitution on human CYP1A1 gene. "nucleotides 13 to 15 are ribonucleotides-other nucleotides are deoxyribonucleotides and the 3'-OH group of the nucleotide at 3'end is protected with amino hexyl group

SEQ ID NO:22: Chimeric oligonucleotide to detect the nucleotide substitution on human CYP1A1 gene. "nucleotides 13 to 15 are ribonucleotides-other nucleotides are deoxyribonucleotides and the 3'-OH group of the nucleotide at 3'end is protected with amino hexyl group

SEQ ID NO:23: Chimeric oligonucleotide primer to amplify a portion of human CYP1A1 gene. "nucleotides 19 to 21 are ribonucleotides-other nucleotides are deoxyribonucleotides, and 5' end is labeled by biotin"

SEQ ID NO:24: Oligonucleotide probe for detecting a portion of human CYP1A1 gene. "5' end is labeled by FITC"

SEQ ID NO:25: Chimeric oligonucleotide to detect the nucleotide substitution on human ALDH2 gene. "nucleotides 16 to 18 are ribonucleotides, nucleotide 20 is inosine-other nucleotides are deoxyribonucleotides and the 3'-OH group of the nucleotide at 3'end is protected with amino hexyl group.

SEQ ID NO:26: Chimeric oligonucleotide to detect the nucleotide substitution on human ALDH2 gene. "nucleotides 16 to 18 are ribonucleotides-other nucleotides are deoxyribonucleotides and the 3'-OH group of the nucleotide at 3'end is protected with amino hexyl group

SEQ ID NO:27: Chimeric oligonucleotide primer to amplify a portion of human ALDH2 gene. "nucleotides 16 to 18 are ribonucleotides-other nucleotides are deoxyribonucleotides, and 5' end is labeled by biotin"

SEQ ID NO:28: Oligonucleotide probe for detecting a portion of human ALDH2 gene. "5' end is labeled by FITC"

SEQ ID NO:29: Chimeric oligonucleotide primer to amplify a portion of human GSTT1 gene. "nucleotides 19 to 21 are ribonucleotides-other nucleotides are deoxyribonucleotides"

SEQ , ID NO:30: Chimeric oligonucleotide primer to amplify a portion of human GSTT1 gene. "nucleotides 19 to 21 are ribonucleotides-other nucleotides are deoxyribonucleotides"

SEQ ID NO:31: Chimeric oligonucleotide primer to amplify a portion of human GSTT1 gene. "nucleotides 19 to 21 are ribonucleotides-other nucleotides are deoxyribonucleotides, and 5' end is labeled by biotin"

SEQ ID NO:32: Oligonucleotide probe for detecting a portion of human GSTT1 gene. "5' end is labeled by FITC"

SEQ ID NO:33: Designed PCR primer to amplify a portion of human GSTT1 gene.

SEQ ID NO:34: Designed PCR primer to amplify a portion of human GSTT1 gene.

SEQ ID NO:35: Chimeric oligonucleotide primer to amplify a portion of human beta-globin gene. "nucleotides 19 to 21 are ribonucleotides-other nucleotides are deoxyribonucleotides"

SEQ ID NO:36: Chimeric oligonucleotide primer to amplify a portion of human beta-globin gene. "nucleotides 20 to 22 are ribonucleotides-other nucleotides are deoxyribonucleotides"

SEQ ID NO:37: Chimeric oligonucleotide primer to amplify a portion of human beta-globin gene. "nucleotides 21 to 23 are ribonucleotides-other nucleotides are deoxyribonucleotides"

SEQ ID NO:38: Chimeric oligonucleotide primer to amplify a portion of human beta-globin gene. "nucleotides 21 to 23 are ribonucleotides-other nucleotides are deoxyribonucleotides"

SEQ ID NO:39: Chimeric oligonucleotide primer to amplify a portion of human beta-globin gene. "nucleotides 20 to 22 are ribonucleotides-other nucleotides are deoxyribonucleotides"

SEQ ID NO:40: Chimeric oligonucleotide primer to amplify a portion of human beta-globin gene. "nucleotides 19 to 21 are ribonucleotides-other nucleotides are deoxyribonucleotides, and 5' end is labeled by biotin"

SEQ ID NO:41: Chimeric oligonucleotide primer to amplify a portion of human beta-globin gene. "nucleotides 21 to 23 are ribonucleotides-other nucleotides are deoxyribonucleotides, and 5' end is labeled by biotin"

SEQ ID NO:42: Chimeric oligonucleotide primer to amplify a portion of human beta-globin gene. "nucleotides 20 to 22 are ribonucleotides-other nucleotides are deoxyribonucleotides, and 5' end is labeled by biotin"

SEQ ID NO:43: Oligonucleotide primer for detecting a portion of human beta-globin gene. "5' end is labeled by FITC"

SEQ ID NO:44: Oligonucleotide primer for detecting a portion of human beta-golbin gene. "5' end is labeled by FITC"

## Claims

1. A method for typing a genetic polymorphism at a specific nucleotide in a human cytochrome gene, G636A in the CYP 2C19 gene or T6235C in the CYP 1A1 gene, the method comprising:

    (1) preparing a reaction mixture by mixing a nucleic acid as a template, deoxyribonucleotide triphosphates, a DNA polymerase having a strand displacement activity, at least one Nucleotide and an RNase H, wherein

        (a) the Nucleotide is modified at the 3' terminus such that extension from the terminus by the action of a DNA polymerase does not take place; and
        (b) the Nucleotide has a nucleotide sequence that is capable of annealing to a region containing the specific nucleotide in the human cytochrome gene; and

    (2) incubating the reaction mixture for a time sufficient for generating a reaction product to extend a nucleic acid containing a region of arbitrary length that contains the specific nucleotide in the human cytochrome gene.

2. The method according to claim 1, wherein the Nucleotide has a nucleotide sequence of SEQ ID NO:14, 15, 21 or 22 or a nucleotide sequence complementary thereto.

3. The method according to claim 1, wherein a primer having a nucleotide sequence of SEQ ID NO:16 or 23 is further used.

4. The method according to claim 1, which further comprises a step of detecting the nucleic acid extended in step (2) using a probe that is capable of hybridizing to the nucleic acid under stringent conditions.

5. The method according to claim 4, wherein the probe has a nucleotide sequence of SEQ ID NO:20 or 24 or a nucleotide sequence complementary thereto.

6. A kit for typing a genetic polymorphism in a human cytochrome gene, which is used for the method of typing a genetic polymorphism in a human cytochrome gene defined by claim 1, and which contains a Nucleotide having a nucleotide sequence of SEQ ID NO:14, 15, 21 or 22 or a nucleotide sequence complementary thereto.

7. The kit according to claim 6, which further contains a primer having a nucleotide sequence of SEQ ID NO:16 or 23.

8. The kit according to claim 7, which further contains a probe having a nucleotide sequence of SEQ ID NO:20 or 24 or a nucleotide sequence complementary thereto.

9. A method for typing a polymorphism in a human glutathione-S-transferase gene, the method comprising:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and
(b) incubating the reaction mixture for a time sufficient for generating a reaction product to amplify a target nucleic acid.

10. The method according to claim 9, wherein the human glutathione-S-transferase gene is the GSTM1 gene or the GSTT1 gene.

11. The method according to claim 9, wherein the primer is a chimeric oligonucleotide primer having a nucleotide sequence of SEQ ID NO:6, 7, 8, 29, 30 or 31 or a nucleotide sequence complementary thereto.

12. The method according to claim 9, which further comprises a step of detecting the nucleic acid amplified in step (b) using a probe that is capable of hybridizing to the nucleic acid under stringent conditions.

13. The method according to claim 12, wherein the probe has a nucleotide sequence of SEQ ID NO:9 or 32 or a nucleotide sequence complementary thereto.

14. A kit for typing a genetic polymorphism in a human glutathione-S-transferase gene, which is used for the method of typing a polymorphism in a human glutathione-S-transferase gene defined by claim 9, and which contains a chimeric oligonucleotide primer having a nucleotide sequence of SEQ ID NO:6, 7, 8, 29, 30 or 31.

15. The kit according to claim 14, which further contains a probe having a nucleotide sequence of SEQ ID NO:9 or 32 or a nucleotide sequence complementary thereto.

16. A method for typing a genetic polymorphism at a specific nucleotide in a human aldehyde dehydrogenase gene, Glu487Lys in the ALDH2 gene, the method comprising:

(1) preparing a reaction mixture by mixing a nucleic acid as a template, deoxyribonucleotide triphosphates, a DNA polymerase having a strand displacement activity, at least one Nucleotide and an RNase H, wherein

(a) the Nucleotide is modified at the 3' terminus such that extension from the terminus by the action of a DNA polymerase does not take place; and
(b) the Nucleotide has a nucleotide sequence that is capable of annealing to a region containing the specific nucleotide in the human aldehyde dehydrogenase gene; and

(2) incubating the reaction mixture for a time sufficient for generating a reaction product to extend a nucleic acid containing a region of arbitrary length that contains the specific nucleotide in the human aldehyde dehydrogenase gene.

17. The method according to claim 16, wherein the Nucleotide has a nucleotide sequence of SEQ ID NO:25 or 26 or a nucleotide sequence complementary thereto.

18. The method according to claim 16, wherein a primer having a nucleotide sequence of SEQ ID NO:27 is further used.

19. The method according to claim 16, which further comprises a step of detecting the nucleic acid extended in step (2) using a probe that is capable of hybridizing to the nucleic acid under stringent conditions.

20. The method according to claim 19, wherein the probe has a nucleotide sequence of SEQ ID NO:28 or a nucleotide sequence complementary thereto.

21. A kit for typing a genetic polymorphism in a human aldehyde dehydrogenase gene, which is used for the method of typing a genetic polymorphism in a human aldehyde dehydrogenase gene defined by claim 16, and which contains a Nucleotide having a nucleotide sequence of SEQ ID NO:25 or 26 or a nucleotide sequence complementary thereto.

**22.** The kit according to claim 21, which further contains a primer having a nucleotide sequence of SEQ ID NO:27.

**23.** The kit according to claim 21, which further contains a probe having a nucleotide sequence of SEQ ID NO:28 or a nucleotide sequence complementary thereto.

**24.** A method for typing genetic polymorphisms, wherein plural target nucleic acids are detected in parallel, the method comprising:

(1) preparing a reaction mixture by mixing nucleic acids as templates, deoxyribonucleotide triphosphates, a DNA polymerase having a strand displacement activity, at least two Nucleotides and an RNase H, wherein

(a) each Nucleotide is modified at the 3' terminus such that extension from the terminus by the action of a DNA polymerase does not take place; and
(b) each Nucleotide has a nucleotide sequence that is capable of annealing to a region containing a specific nucleotide in the plural target nucleic acids; and

(2) incubating the reaction mixture for a time sufficient for generating reaction products to extend nucleic acids each containing a region of arbitrary length that contains the specific nucleotide.

**25.** A method for typing genetic polymorphisms, wherein plural target nucleic acids are detected in parallel, the method comprising:

(a) preparing a reaction mixture by mixing nucleic acids as templates, deoxyribonucleotide triphosphates, a DNA polymerase having a strand displacement activity, at least two primers and an RNase H, wherein each primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and
(b) incubating the reaction mixture for a time sufficient for generating reaction products to amplify target nucleic acids.

**26.** A method for typing genetic polymorphisms, wherein plural target nucleic acids are detected in parallel, the method comprising:

(A) detecting at least one target nucleic acid according to a method comprising:

(1) preparing a reaction mixture by mixing a nucleic acid as a template, deoxyribonucleotide triphosphates, a DNA polymerase having a strand displacement activity, at least one Nucleotide and an RNase H, wherein

(a) the Nucleotide is modified at the 3' terminus such that extension from the terminus by the action of a DNA polymerase does not take place; and
(b) the Nucleotide has a nucleotide sequence that is capable of annealing to a region containing a specific nucleotide in the plural target nucleic acids; and

(2) incubating the reaction mixture for a time sufficient for generating a reaction product to extend a nucleic acid containing a region of arbitrary length that contains the specific nucleotide; and

(B) detecting a target nucleic acid that is different from the target nucleic acid in (A) according to a method comprising:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains a ribonucleotide as well as at least one selected from the group consisting of a deoxyribonucleotide and a nucleotide analog, the ribonucleotide being positioned at the 3' terminus or on the 3'-terminal side of the primer; and
(b) incubating the reaction mixture for a time sufficient for generating a reaction product to amplify a target nucleic acid.

**27.** The method according to any one of claims 24 to 26, wherein at least one of the target nucleic acids is a nucleic acid as an internal standard.

**28.** The method according to claim 27, wherein the nucleic acid as an internal standard is detected using at least two primers selected from the group consisting of primers having nucleotide sequences of SEQ ID NOS:35-42.

**29.** The method according to claim 28, wherein the nucleic acid as an internal standard is detected further using a probe having a nucleotide sequence of SEQ ID NO:43 or 44 or a nucleotide sequence complementary thereto.

**30.** A kit for the method of typing genetic polymorphisms defined by any one of claims 24 to 26.

**31.** The kit according to claim 30, which contains at least two primers selected from the group consisting of primers having nucleotide sequences of SEQ ID NOS:35-42.

**32.** The kit according to claim 31, which further contains a probe having a nucleotide sequence of SEQ ID NO:43 or 44 or a nucleotide sequence complementary thereto.

Fig. 1

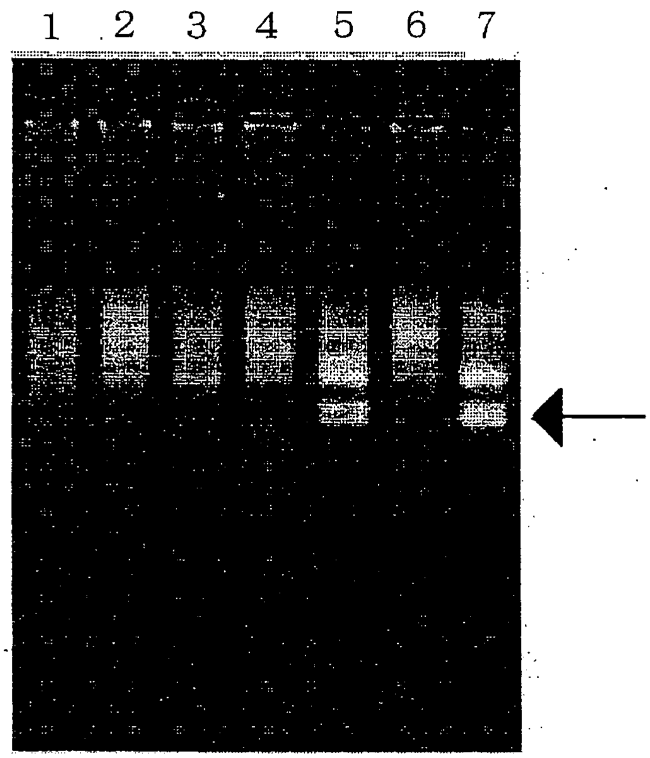

Fig. 2

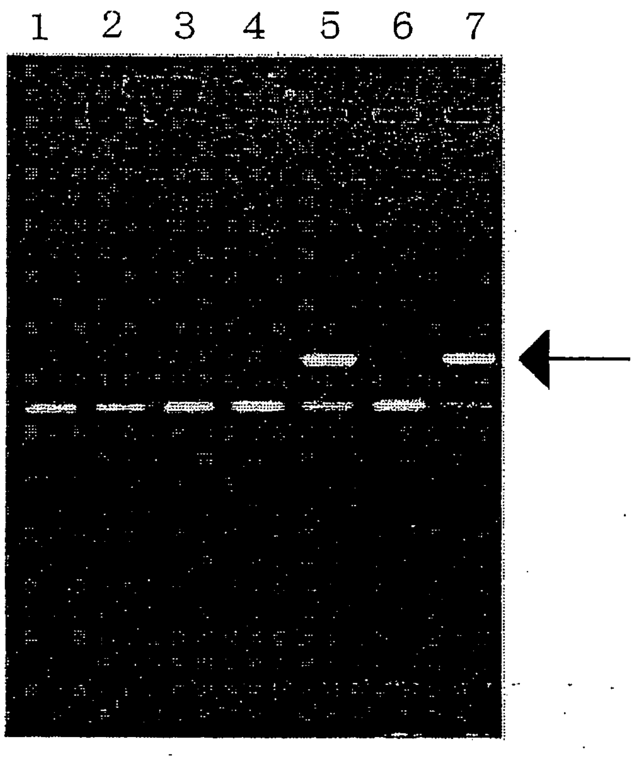

Fig. 3

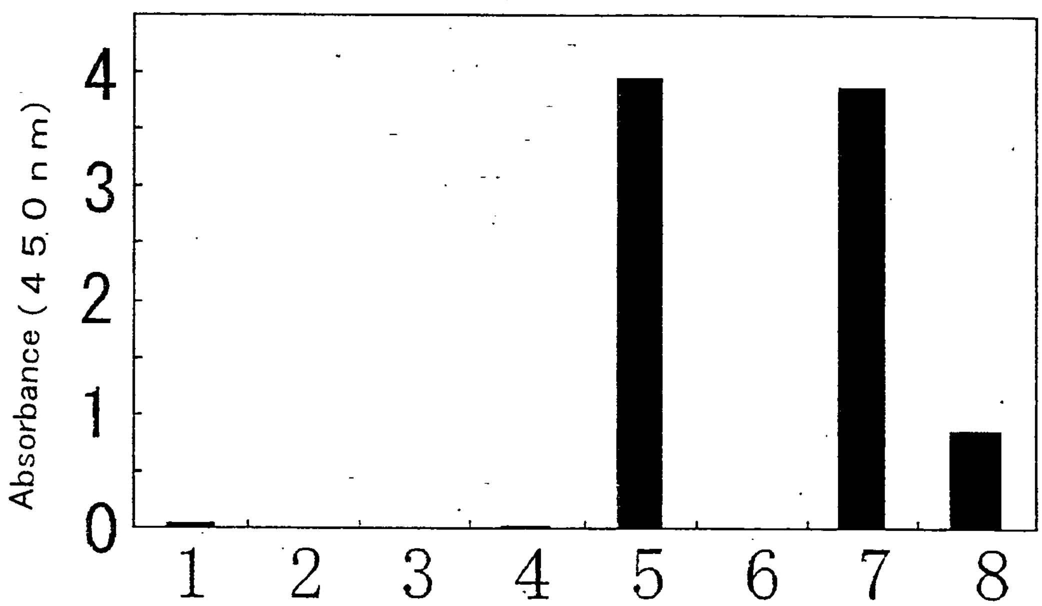

Fig. 4

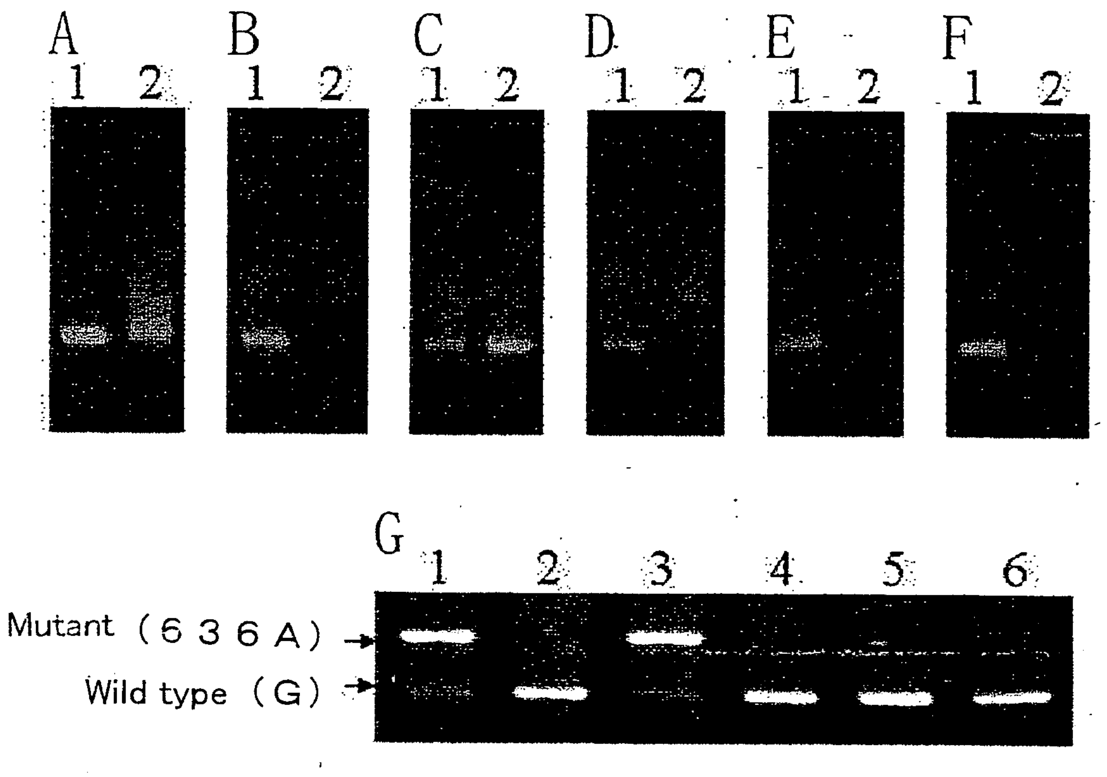

Fig. 5

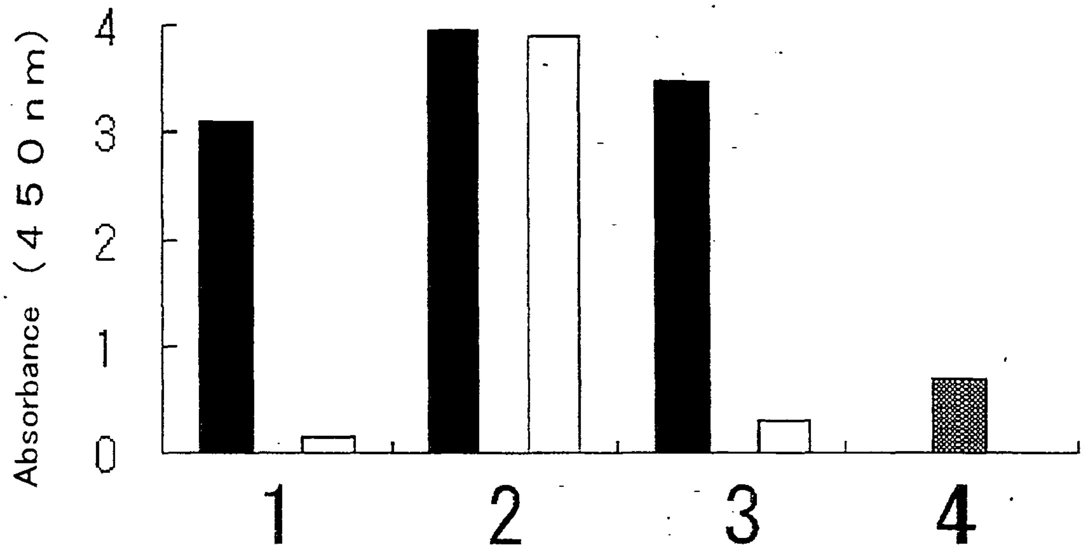

Fig. 6

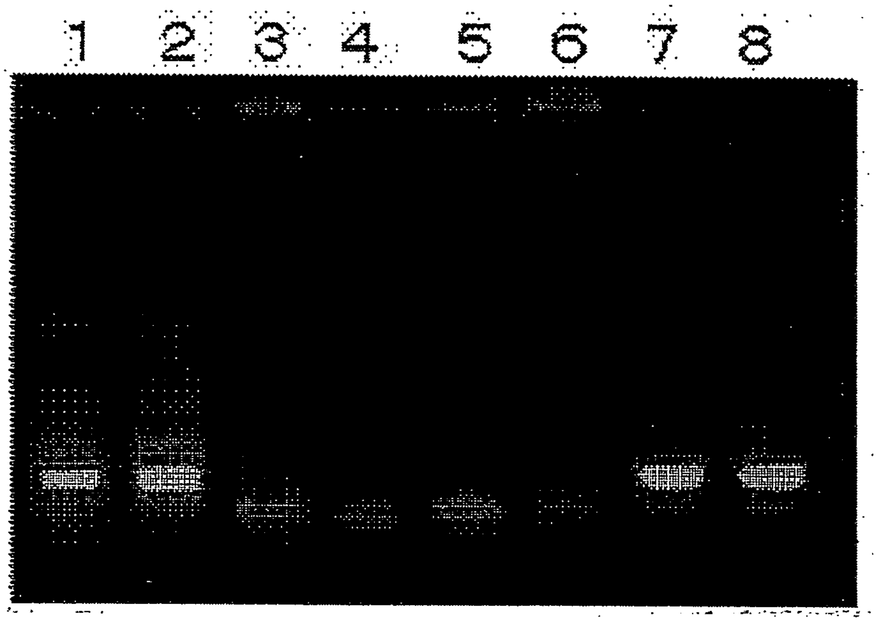

Fig. 7

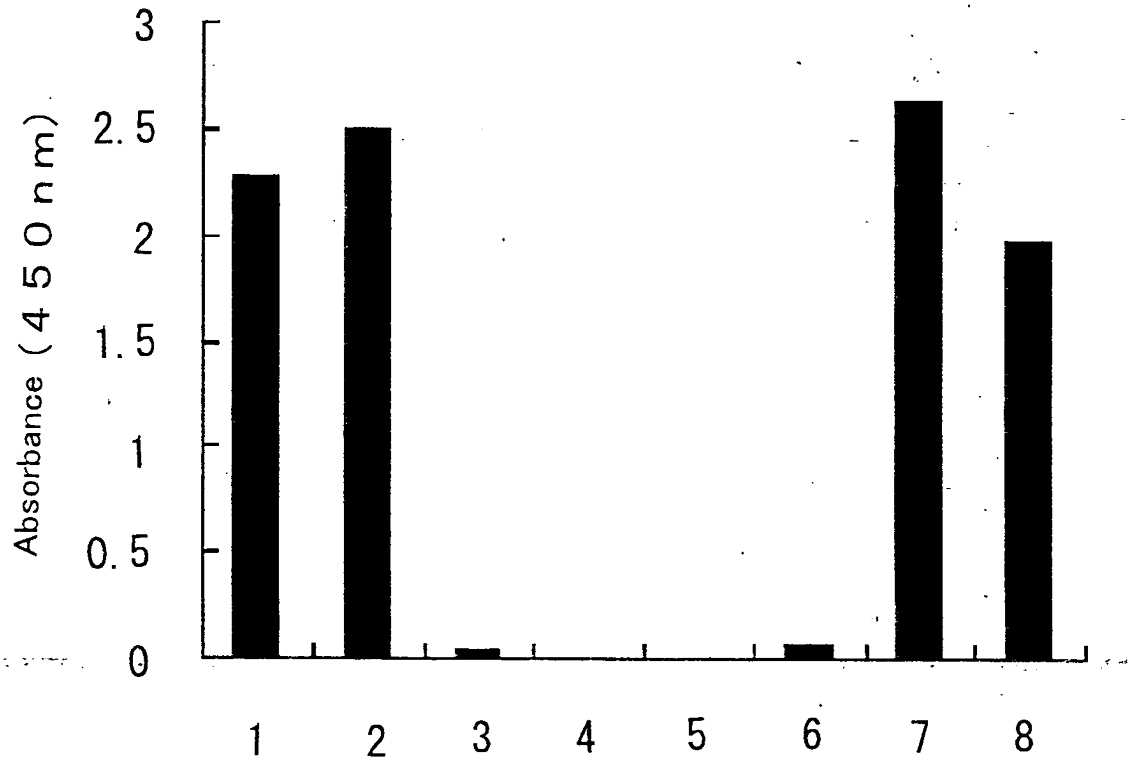

Fig. 8

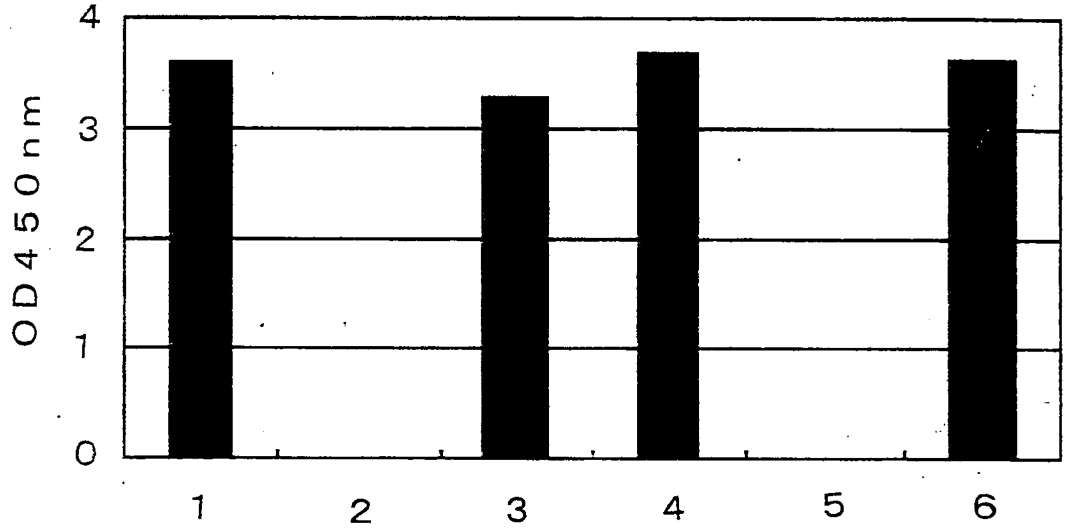

Fig. 9

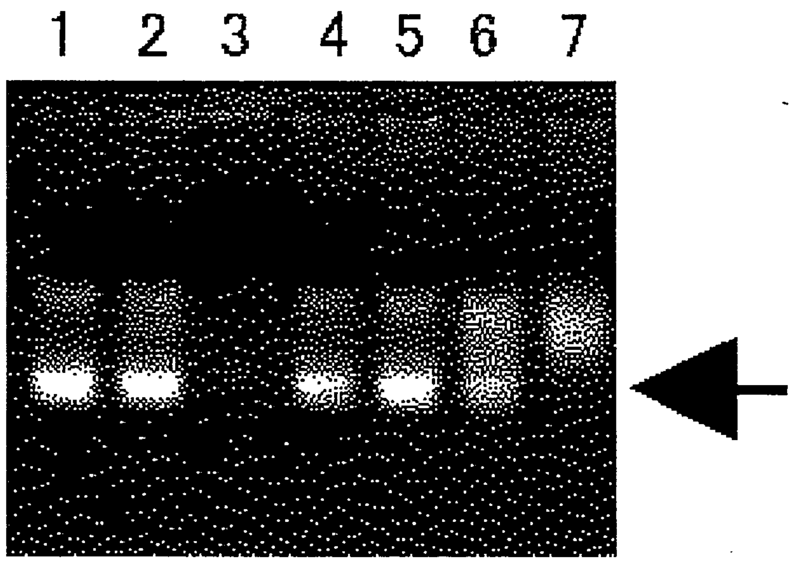

Fig. 10

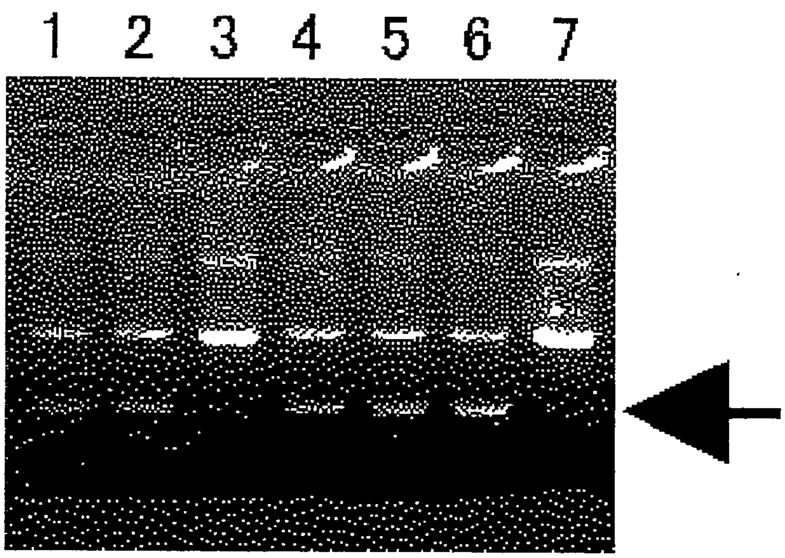

Fig. 11

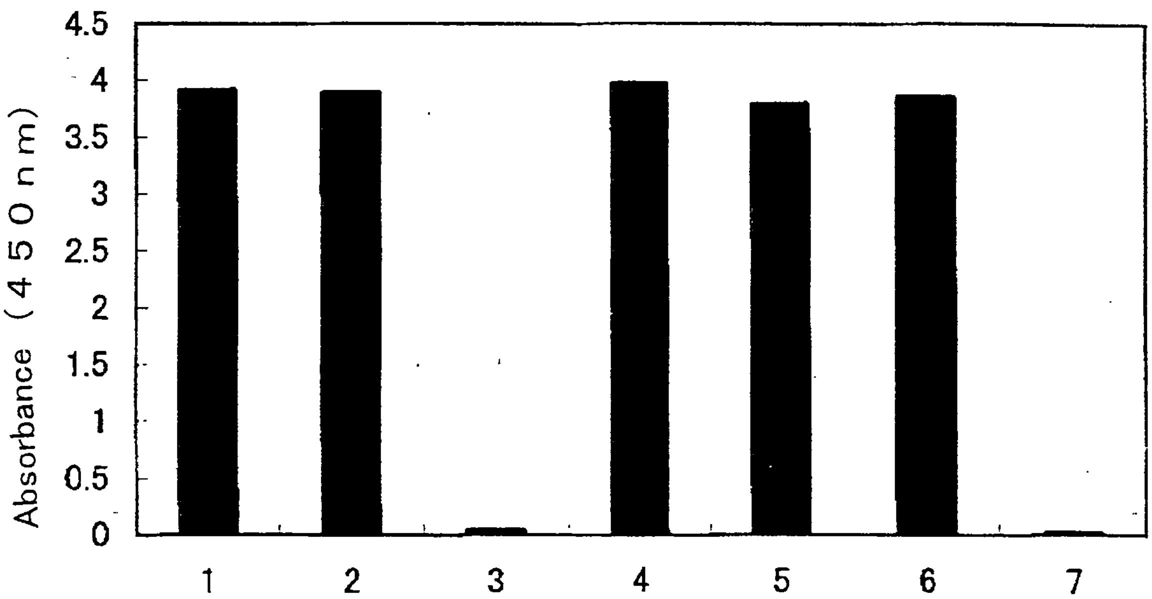

Fig. 12

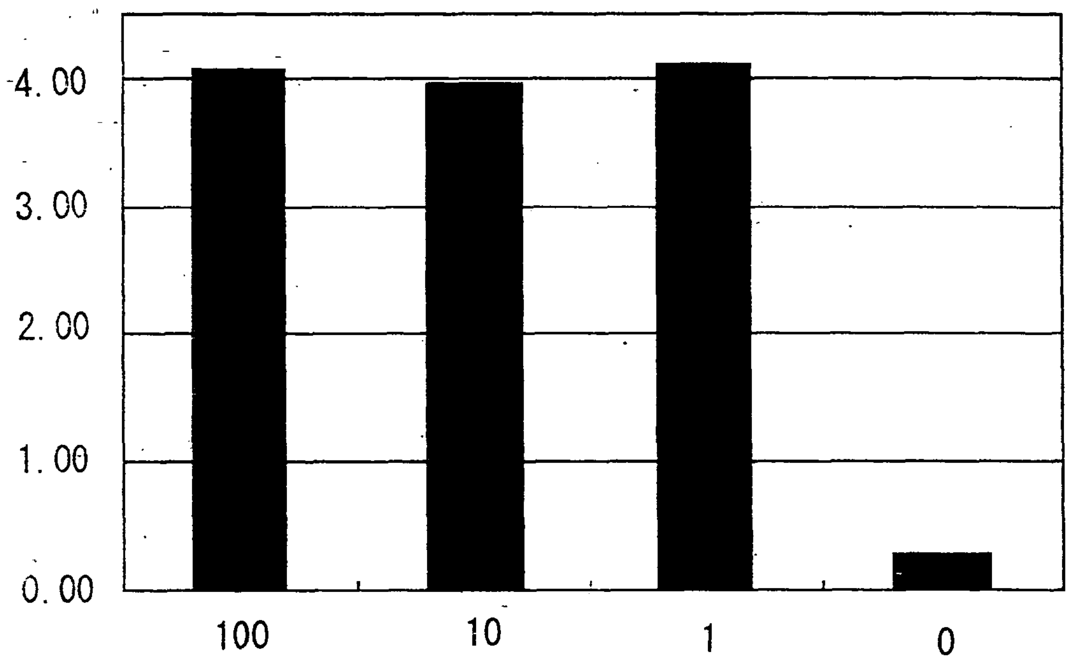

Fig. 13

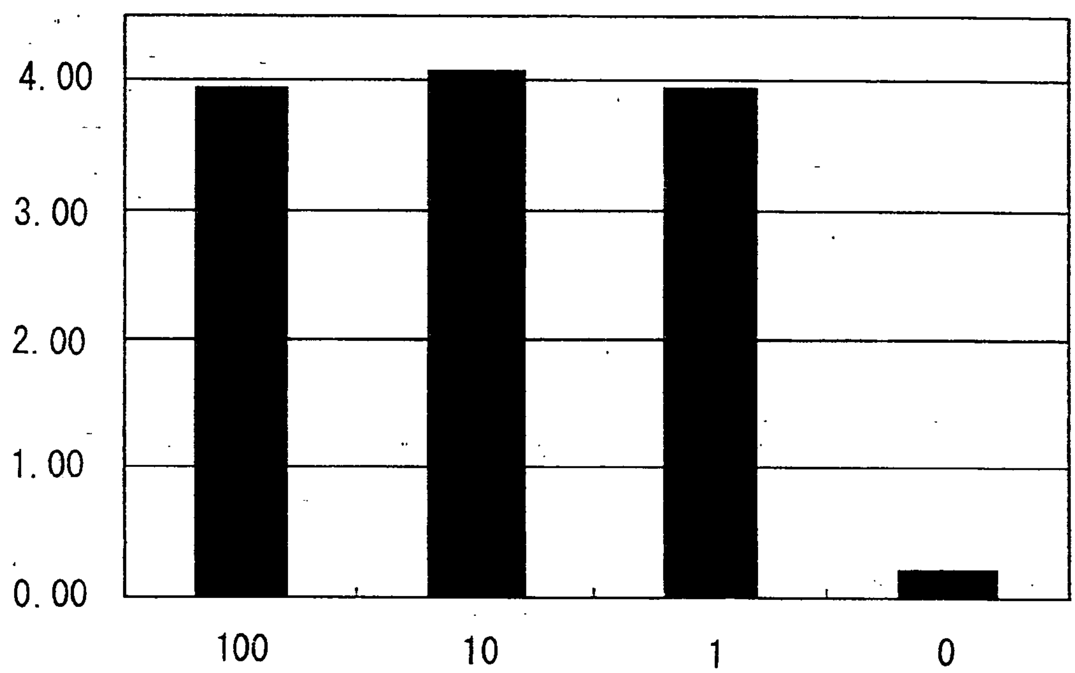

Fig. 14

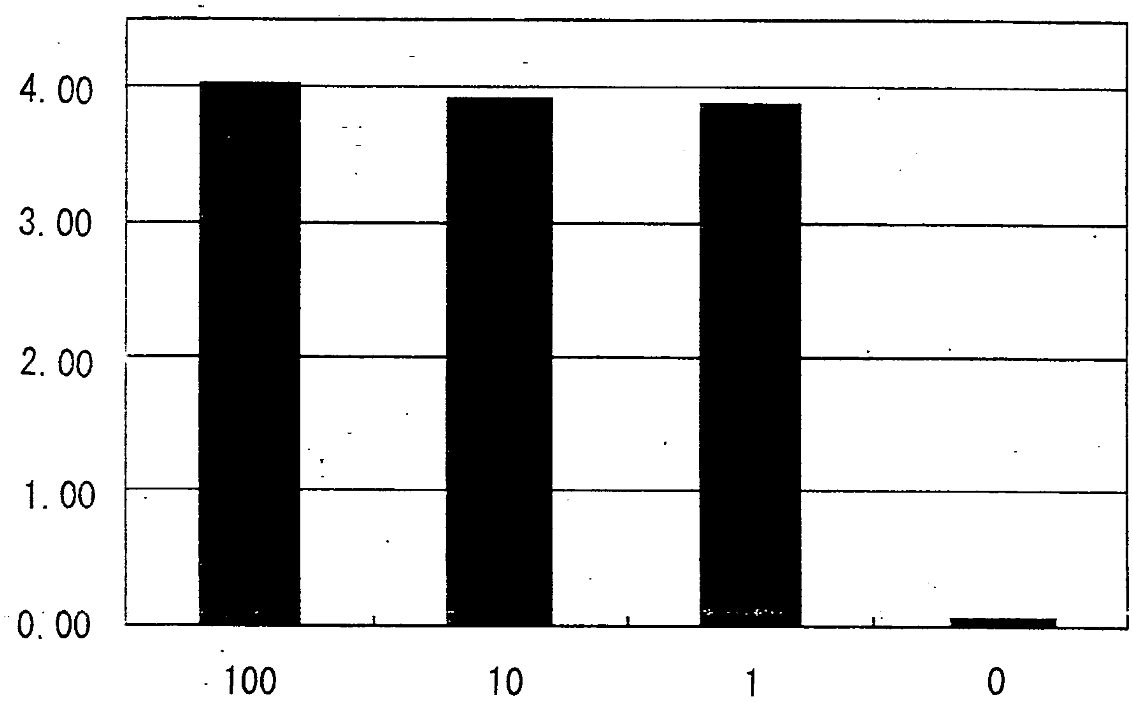

Fig. 15

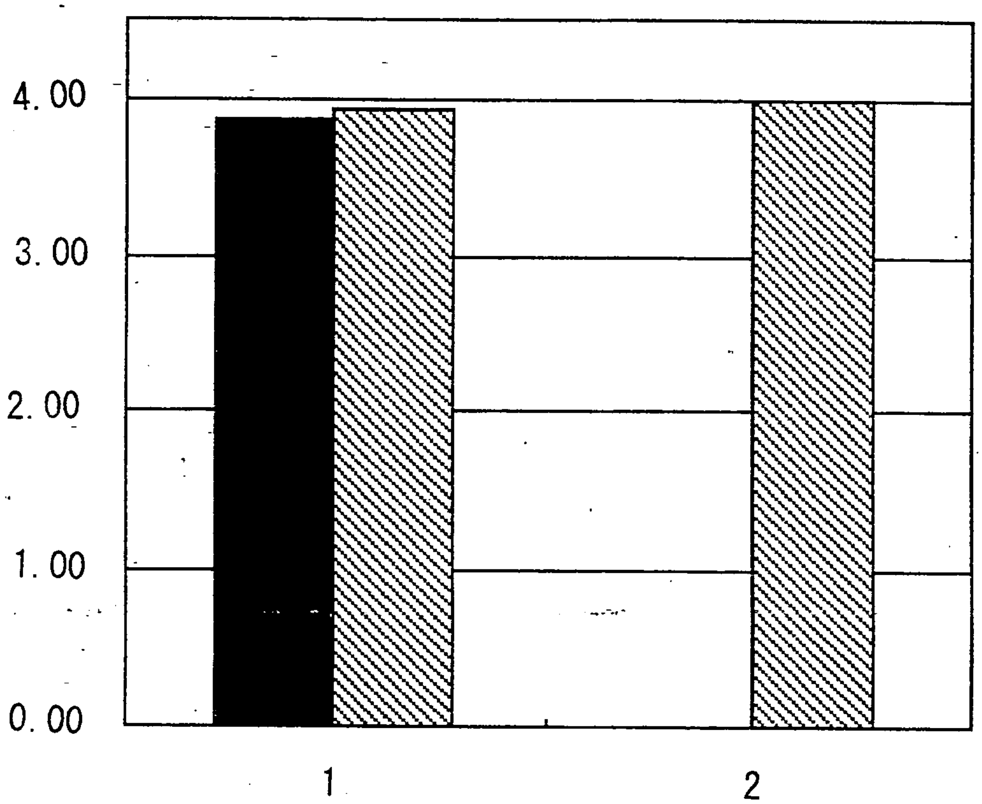

Fig. 16

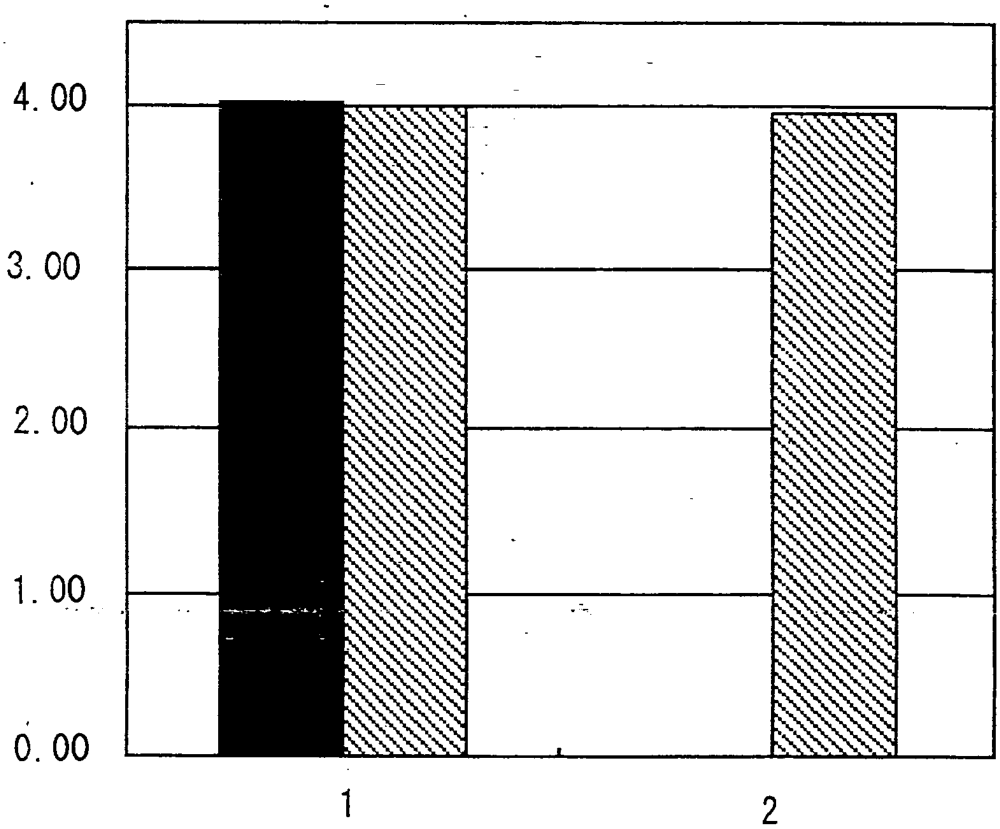

Fig. 17

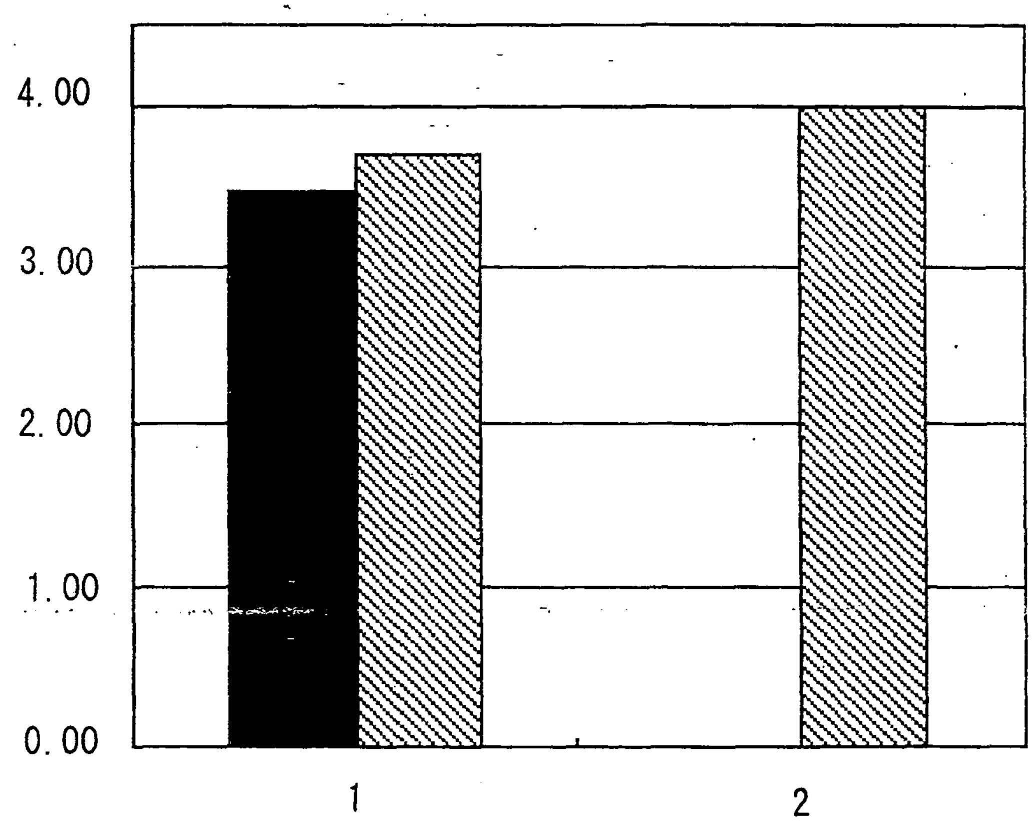

Fig. 18

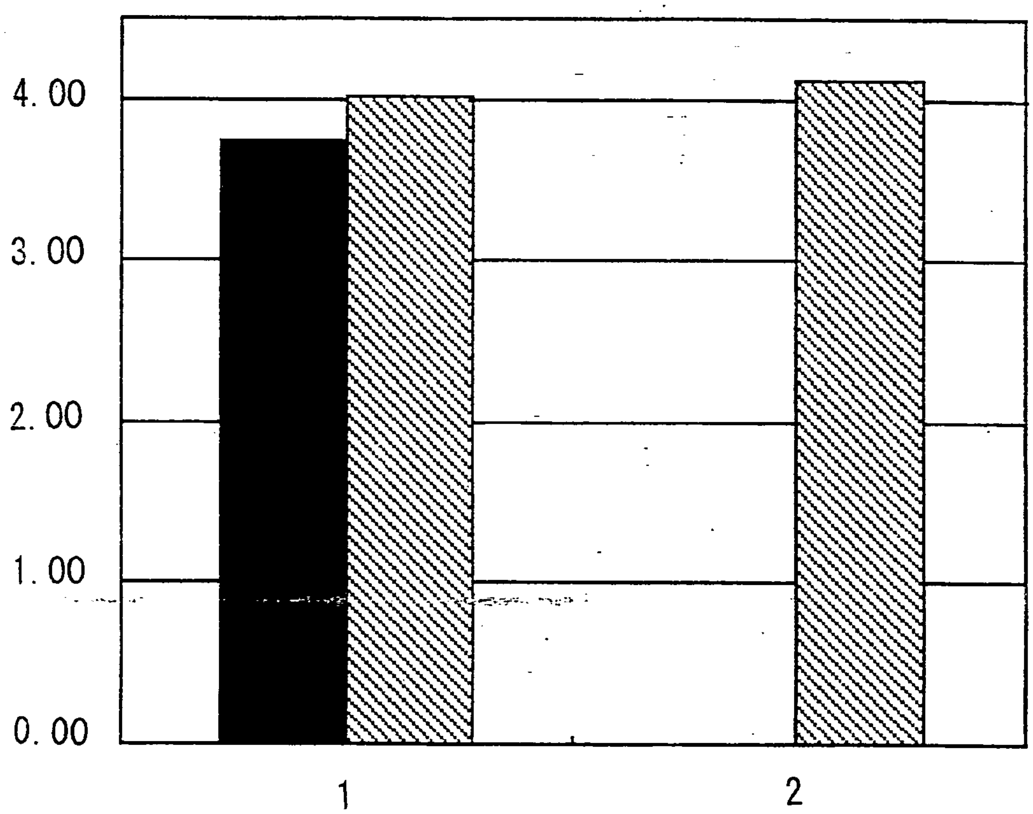

Fig. 19

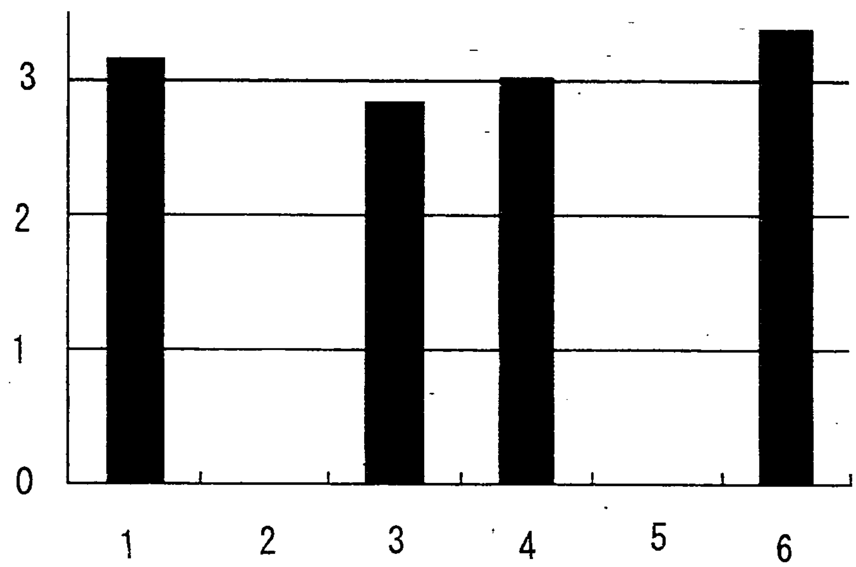

Fig. 20

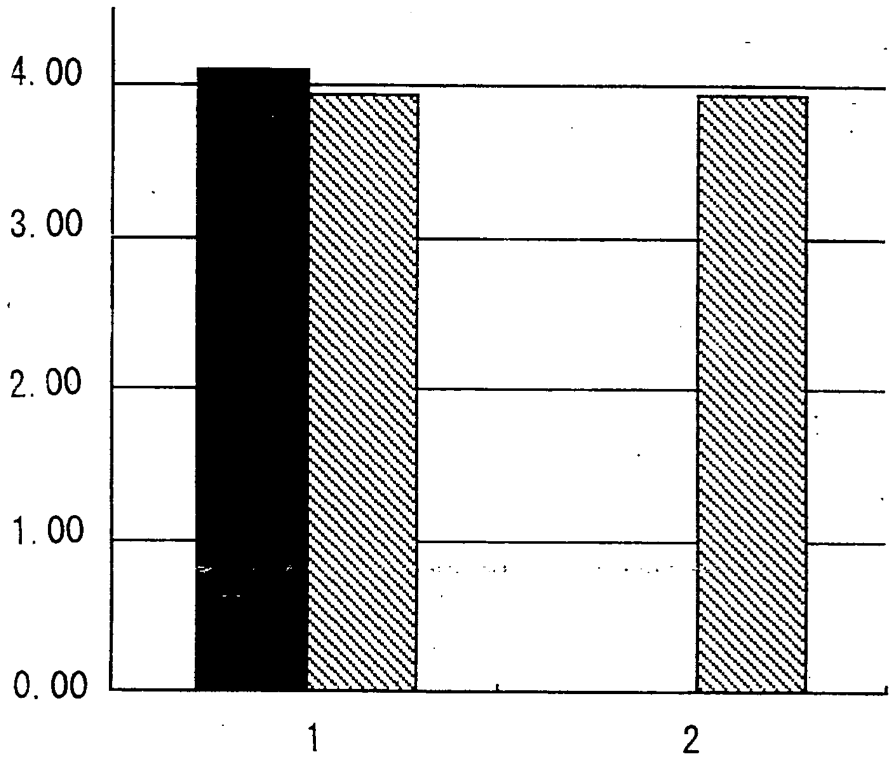

## INTERNATIONAL SEARCH REPORT

International application No.
PCT/JP03/06804

A. CLASSIFICATION OF SUBJECT MATTER
Int.Cl[7] C12N15/00, C12Q1/68, G01N33/50

According to International Patent Classification (IPC) or to both national classification and IPC

B. FIELDS SEARCHED

Minimum documentation searched (classification system followed by classification symbols)
Int.Cl[7] C12N15/00-90, C12Q1/00-70, G01N33/00-98

Documentation searched other than minimum documentation to the extent that such documents are included in the fields searched

Electronic data base consulted during the international search (name of data base and, where practicable, search terms used)
MEDLINE(STN), WPI(DIALOG), BIOSIS(DIALOG), GenBank/EMBL/DDBJ/Geneseq

C. DOCUMENTS CONSIDERED TO BE RELEVANT

| Category* | Citation of document, with indication, where appropriate, of the relevant passages | Relevant to claim No. |
|---|---|---|
| Y | TAKARA HOLDINGS INC. News Release 2001 nen "Shinki SNP Typing-ho (UCAN-ho) o Kaihotsu", 05 November, 2001 (05.11.01) [retrieved on 2003-07-18] Retrieved from the Internet: <URL:http://www.takara.co.jp/new/2001/index.htm> | 1-32 |
| P,Y | WO 02/64833 A1 (TAKARA BIO INC. et al.), 22 August, 2002 (22.08.02), & WO 02/101041 A1 | 1-32 |
| Y | De Morais SM. et al., "Identification of a new genetic defect responsible for the polymorphism of (S)-mephenytoin metabolism in Japanese." Molecular Pharmacology, Vol.46, No.4, (1994), pages 594 to 598 | 1-8 |

☒ Further documents are listed in the continuation of Box C.   ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 July, 2003 (18.07.03) | 12 August, 2003 (12.08.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/06804

C (Continuation). DOCUMENTS CONSIDERED TO BE RELEVANT

| Category* | Citation of document, with indication, where appropriate, of the relevant passages | Relevant to claim No. |
|---|---|---|
| P,Y | WO 02/04683 A2 (GENELINK INC.), 17 October, 2002 (17.10.02), & US 2002/0146698 A1 & US 2003/0073612 A1 | 9-15 |
| Y | JP 2002-058483 A (OTSUKA SEIYAKU KOGYO KABUSHIKI KAISHA), 26 February, 2002 (26.02.02), (Family: none) | 9-15 |
| Y | WO 02/80755 A2 (UNIV. VANDERBILT), 17 January, 2002 (17.01.02), (Family: none) | 9-15 |
| Y | McClay JL. et al., "High-throughput single-nucleotide polymorphism genotyping by fluorescent competitive allele-specific polymerase chain reaction (SNiPTag).", Analytical Biochemistry, Vol.301, No.2, 15 February, 2002 (15.02.02), pages 200 to 206. | 16-23 |

Form PCT/ISA/210 (continuation of second sheet) (July 1998)

SEQUENCE LISTING

<110> TAKARA BIO INC.

<120> Typing method for genetic polymorphism

<130> 38-81

<150> JP 2002-158853

<151> 2002-05-31

<150> JP 2002-204143

<151> 2002-07-12

<150> JP 2002-211813

<151> 2002-07-19

<150> JP 2002-310862

<151> 2002-10-25

<150> JP 2002-335830

<151> 2002-11-19

<160> 44

<210> 1

<211> 626

<212> DNA

<213> Archaeoglobus fulgidus

<400> 1

atgaaggcag gcatcgatga ggctggaaag ggctgcgtca tcggcccact ggttgttgca 60
ggagtggctt gcagcgatga ggataggctg agaaagcttg gtgtgaaaga ctccaaaaag 120
ctaagtcagg ggaggagaga ggaactagcc gaggaaataa ggaaaatctg cagaacggag 180
gttttgaaag tttctcccga aaatctcgac gaaaggatgg ctgctaaaac cataaacgag 240
attttgaagg agtgctacgc tgaaataatt ctcaggctga agccggaaat tgcttatgtt 300
gacagtcctg atgtgattcc cgagagactt tcgagggagc ttgaggagat tacggggttg 360
agagttgtgg ccgagcacaa ggcggacgag aagtatcccc tggtagctgc ggcttcaatc 420
atcgcaaagg tggaaaggga gcgggagatt gagaggctga aagaaaaatt cggggatttc 480
ggcagcggct atgcgagcga tccgaggaca agagaagtgc tgaaggagtg gatagcttca 540
ggcagaattc cgagctgcgt gagaatgcgc tggaagacgg tgtcaaatct gaggcagaag 600
acgcttgacg atttctaaac gaaacc 626

<210> 2
<211> 30
<212> DNA
<213> Artificial Sequence

<220>
<223> PCR primer AfuNde for cloning a gene encoding a polypeptide having a RNaseHII activity from Archaeoglobus fulgidus

<400> 2

aagctgggtt tcatatgaag gcaggcatcg 30

<210> 3

<211> 30

<212> DNA

<213> Artificial Sequence

<220>

<223> PCR primer AfuBam for cloning a gene encoding a polypeptide having a RNaseHII activity from Archaeoglobus fulgidus

<400> 3

tggtaataac ggatccgttt agaaatcgtc 30

<210> 4

<211> 638

<212> DNA

<213> Archaeoglobus fulgidus

<400> 4

catatgaagg caggcatcga tgaggctgga aagggctgcg tcatcggccc actggttgtt 60

gcaggagtgg cttgcagcga tgaggatagg ctgagaaagc ttggtgtgaa agactccaaa 120

aagctaagtc aggggaggag agaggaacta gccgaggaaa taaggaaaat ctgcagaacg 180

gaggttttga aagtttctcc cgaaaatctc gacgaaagga tggctgctaa aaccataaac 240

gagattttga aggagtgcta cgctgaaata attctcaggc tgaagccgga aattgcttat 300

gttgacagtc ctgatgtgat tcccgagaga ctttcgaggg agcttgagga gattacgggg 360

ttgagagttg tggccgagca caaggcggac gagaagtatc ccctggtagc tgcggcttca 420

atcatcgcaa aggtggaaag ggagcgggag attgagaggc tgaaagaaaa attcggggat 480

ttcggcagcg gctatgcgag cgatccgagg acaagagaag tgctgaagga gtggatagct 540

tcaggcagaa ttccgagctg cgtgagaatg cgctggaaga cggtgtcaaa tctgaggcag 600

aagacgcttg acgatttcta aacggatccc cgggtacc 638

<210> 5

<211> 205

<212> PRT

<213> Archaeoglobus fulgidus

<400> 5

Met Lys Ala Gly Ile Asp Glu Ala Gly Lys Gly Cys Val Ile Gly
1 5 10 15

Pro Leu Val Val Ala Gly Val Ala Cys Ser Asp Glu Asp Arg Leu
20 25 30

Arg Lys Leu Gly Val Lys Asp Ser Lys Lys Leu Ser Gln Gly Arg
35 40 45

Arg Glu Glu Leu Ala Glu Glu Ile Arg Lys Ile Cys Arg Thr Glu
50 55 60

Val Leu Lys Val Ser Pro Glu Asn Leu Asp Glu Arg Met Ala Ala
65 70 75

Lys Thr Ile Asn Glu Ile Leu Lys Glu Cys Tyr Ala Glu Ile Ile
80 85 90

Leu Arg Leu Lys Pro Glu Ile Ala Tyr Val Asp Ser Pro Asp Val
95 100 105

Ile Pro Glu Arg Leu Ser Arg Glu Leu Glu Glu Ile Thr Gly Leu
110 115 120

Arg Val Val Ala Glu HisLys Ala Asp Glu Lys Tyr Pro Leu Val
125 130 135

Ala Ala Ala Ser Ile Ile Ala Lys Val Glu Arg Glu Arg Glu Ile
140 145 150

Glu Arg Leu Lys Glu Lys Phe Gly Asp Phe Gly Ser Gly Tyr Ala

155 160 165

Ser Asp Pro Arg Thr Arg Glu Val Leu Lys Glu Trp Ile Ala Ser

170 175 180

Gly Arg Ile Pro Ser Cys Val Arg Met Arg Trp Lys Thr Val Ser

185 190 195

Asn Leu Arg Gln Lys Thr Leu Asp Asp Phe

200 205

<210> 6

<211> 22

<212> DNA

<213> Artificial Sequence

<220>

<223> Chimeric oligonucleotide primer to amplify a portion of human GSTM1 gene. "nucleotides 20 to 22 are ribonucleotides-other nucleotides are deoxyribonucleotides"

<400> 6

gagtctgtgt tttgtgggtg gc 22

<210> 7

<211> 23

<212> DNA

<213> Artificial Sequence

<220>

<223> Chimeric oligonucleotide primer to amplify a portion of human GSTM1 gene. “nucleotides 21 to 23 are ribonucleotides-other nucleotides are deoxyribonucleotides”

<400> 7

cagatcatgc ccagctgcat aug 23

<210> 8

<211> 23

<212> DNA

<213> Artificial Sequence

<220>

<223> Chimeric oligonucleotide primer to amplify a portion of human GSTM1 gene. “nucleotides 21 to 23 are ribonucleotides-other nucleotides are deoxyribonucleotides, and 5' end is labeled by biotin”

<400> 8

cagatcatgc ccagctgcat aug 23

<210> 9

<211> 15

<212> DNA

<213> Artificial Sequence

<220>

<223> Oligonucleotide probe for detecting a portion of human GSTM1 gene. "5' end is labeled by FITC"

<400> 9

agacagaaga ggaga 15

<210> 10

<211> 20

<212> DNA

<213> Artificial Sequence

<220>

<223> Designed PCR primer to amplify a portion of human GSTM1 gene

<400> 10

gtggcaggtg gggagacaga 20

<210> 11

<211> 20

<212> DNA

<213> Artificial Sequence

<220>

<223> Designed PCR primer to amplify a portion of human GSTM1 gene

<400> 11

tgtccatggt ctggttctcc 20

<210> 12

<211> 21

<212> DNA

<213> Artificial Sequence

<220>

<223> Designed PCR primer to amplify a portion of human GSTM1 gene

<400> 12

ctgccctact tgattgatgg g 21

<210> 13

<211> 21

<212> DNA

<213> Artificial Sequence

<220>

<223> Designed PCR primer to amplify a portion of human GSTM1 gene

<400> 13

ctggattgta gcagatcatg c 21

<210> 14

<211> 18

<212> DNA

<213> Artificial Sequence

<220>

<223> Chimeric oligonucleotide to detect the nucleotide substitution on human CYP2C19 gene. “nucleotides 13 to 15 are ribonucleotides-other nucleotides are deoxyribonucleotides and the 3'-OH group of the nucleotide at 3'end is protected with amino hexyl group”

<400> 14

gtaagcaccc ccuggatc 18

<210> 15

<211> 18

<212> DNA

<213> Artificial Sequence

<220>

<223> Chimeric oligonucleotide to detect the nucleotide substitution on human CYP2C19 gene. “nucleotides 13 to 15 are ribonucleotides-other nucleotides are deoxyribonucleotides and the 3'-OH group of the nucleotide at 3'end is protected with amino hexyl group

<400> 15

gtaagcaccc ccugaatc 18

<210> 16

<211> 21

<212> DNA

<213> Artificial Sequence

<220>

<223> Chimeric oligonucleotide primer to amplify a portion of human CYP2C19 gene. “nucleotides 19 to 21 are ribonucleotides-other nucleotides are deoxyribonucleotides”

<400> 16

ttggtcaata tagaatttug g 21

<210> 17

<211> 22

<212> DNA

<213> Artificial Sequence

<220>

<223> Designed PCR primer to amplify a portion of human CYP2C19 gene

<400> 17

tattatctgt taactaatat ga 22

<210> 18

<211> 20

<212> DNA

<213> Artificial Sequence

<220>

<223> Designed PCR primer to amplify a portion of human CYP2C19 gene

<400> 18

acttcagggc ttggtcaata 20

<210> 19

<211> 21

<212> DNA

<213> Artificial Sequence

<220>

<223> Chimeric oligonucleotide primer to amplify a portion of human CYP2C19 gene. “nucleotides 19 to 21 are ribonucleotides-other nucleotides are deoxyribonucleotides, and 5' end is labeled by biotin”

<400> 19

ttggtcaata tagaatttug g 21

<210> 20

<211> 20

<212> DNA

<213> Artificial Sequence

<220>

<223> Oligonucleotide probe for detecting a portion of human CYP2C19 gene. "5' end is labeled by FITC"

<400> 20

caagtttttt gcttcctgag 20

<210> 21

<211> 18

<212> DNA

<213> Artificial Sequence

<220>

<223> Chimeric oligonucleotide to detect the nucleotide substitution on human CYP1A1 gene. “nucleotides 13 to 15 are ribonucleotides-other nucleotides are deoxyribonucleotides and the 3'-OH group of the nucleotide at 3'end is protected with amino hexyl group

<400> 21

aatcgtgtga gcccggga 18

<210> 22

<211> 18

<212> DNA

<213> Artificial Sequence

<220>

<223> Chimeric oligonucleotide to detect the nucleotide substitution on human CYP1A1 gene. “nucleotides 13 to 15 are ribonucleotides-other nucleotides are deoxyribonucleotides and the 3'-OH group of the nucleotide at 3'end is protected with amino hexyl group

<400> 22

atcgtgtgag cccaggag 18

<210> 23

<211> 21

<212> DNA

<213> Artificial Sequence

<220>

<223> Chimeric oligonucleotide primer to amplify a portion of human CYP1A1 gene. “nucleotides 19 to 21 are ribonucleotides-other nucleotides are deoxyribonucleotides, and 5' end is labeled by biotin”

<400> 23

agactctatt ttttgagaca g 21

<210> 24

<211> 17

<212> DNA

<213> Artificial Sequence

<220>

<223> Oligonucleotide probe for detecting a portion of human CYP1A1 gene. "5' end is labeled by FITC"

<400> 24

tggaggttac agtgaaa 17

<210> 25

<211> 21

<212> DNA

<213> Artificial Sequence

<220>

<223> Chimeric oligonucleotide to detect the nucleotide substitution on human ALDH2 gene. “nucleotides 16 to 18 are ribonucleotides, nucleotide 20 is inosine-other nucleotides are deoxyribonucleotides and the 3'-OH group of the nucleotide at 3'end is protected with amino hexyl group

<220>

<221> modified_base

<222> 20

<223> i

<400> 25

ctcacagttt tcactucagn g 21

<210> 26

<211> 21

<212> DNA

<213> Artificial Sequence

<220>

<223> Chimeric oligonucleotide to detect the nucleotide substitution on human ALDH2 gene. “nucleotides 16 to 18 are ribonucleotides-other nucleotides are deoxyribonucleotides and the 3'-OH group of the nucleotide at 3'end is protected with

amino hexyl group

<400> 26
actcacagtt ttcacuuuag t 21

<210> 27
<211> 18
<212> DNA
<213> Artificial Sequence

<220>
<223> Chimeric oligonucleotide primer to amplify a portion of human ALDH2 gene. “nucleotides 16 to 18 are ribonucleotides-other nucleotides are deoxyribonucleotides, and 5' end is labeled by biotin”

<400> 27
tcaccctttg gtggcuac 18

<210> 28
<211> 14
<212> DNA
<213> Artificial Sequence

<220>
<223> Oligonucleotide probe for detecting a portion of human ALDH2 gene. "5' end is labeled by FITC"

<400> 28
tgcagcccgt actc 14

<210> 29
<211> 21
<212> DNA
<213> Artificial Sequence

<220>
<223> Chimeric oligonucleotide primer to amplify a portion of human GSTT1 gene.
"nucleotides 19 to 21 are ribonucleotides-other nucleotides are deoxyribonucleotides"

<400> 29
accctgcagt tgctcgagga c 21

<210> 30
<211> 21
<212> DNA
<213> Artificial Sequence

<220>
<223> Chimeric oligonucleotide primer to amplify a portion of human GSTT1 gene.
"nucleotides 19 to 21 are ribonucleotides-other nucleotides are deoxyribonucleotides"

<400> 30
cgtgatggct acgaggtcag c 21

<210> 31

<211> 21

<212> DNA

<213> Artificial Sequence

<220>

<223> Chimeric oligonucleotide primer to amplify a portion of human GSTT1 gene. “nucleotides 19 to 21 are ribonucleotides-other nucleotides are deoxyribonucleotides, and 5' end is labeled by biotin”

<400> 31

accctgcagt tgctcgagga c 21

<210> 32

<211> 15

<212> DNA

<213> Artificial Sequence

<220>

<223> Oligonucleotide probe for detecting a portion of human GSTT1 gene. "5' end is labeled by FITC"

<400> 32

aaggagatgt gagga 15

<210> 33

<211> 23

<212> DNA

<213> Artificial Sequence

<220>

<223> Designed PCR primer to amplify a portion of human GSTT1 gene.

<400> 33

ttccttactg gtcctcacat ctc 23

<210> 34

<211> 20

<212> DNA

<213> Artificial Sequence

<220>

<223> Designed PCR primer to amplify a portion of human GSTT1 gene.

<400> 34

tcaccggatc atggccagca 20

<210> 35

<211> 21

<212> DNA

<213> Artificial Sequence

<220>

<223> Chimeric oligonucleotide primer to amplify a portion of human beta-globin gene. “nucleotides 19 to 21 are ribonucleotides-other nucleotides are deoxyribonucleotides”

<400> 35

ggtggtctac ccttggaccc a 21

<210> 36

<211> 22

<212> DNA

<213> Artificial Sequence

<220>

<223> Chimeric oligonucleotide primer to amplify a portion of human beta-globin gene. “nucleotides 20 to 22 are ribonucleotides-other nucleotides are deoxyribonucleotides”

<400> 36

gccttcacct tagggttgcc ca 22

<210> 37

<211> 23

<212> DNA

<213> Artificial Sequence

<220>

<223> Chimeric oligonucleotide primer to amplify a portion of human beta-globin gene. “nucleotides 21 to 23 are ribonucleotides-other nucleotides are deoxyribonucleotides”

<400> 37
cttggaccca gaggttcttt gag 23

<210> 38
<211> 23
<212> DNA
<213> Artificial Sequence

<220>
<223> Chimeric oligonucleotide primer to amplify a portion of human beta-globin gene.
"nucleotides 21 to 23 are ribonucleotides-other nucleotides are deoxyribonucleotides"

<400> 38
ctttcttgcc atgagccttc acc 23

<210> 39
<211> 22
<212> DNA
<213> Artificial Sequence

<220>
<223> Chimeric oligonucleotide primer to amplify a portion of human beta-globin gene.
"nucleotides 20 to 22 are ribonucleotides-other nucleotides are deoxyribonucleotides"

<400> 39
gccatgagcc ttcaccttag gg 22

<210> 40

<211> 21

<212> DNA

<213> Artificial Sequence

<220>

<223> Chimeric oligonucleotide primer to amplify a portion of human beta-globin gene. “nucleotides 19 to 21 are ribonucleotides-other nucleotides are deoxyribonucleotides, and 5’ end is labeled by biotin”

<400> 40

ggtggtctac ccttggaccc a 21

<210> 41

<211> 23

<212> DNA

<213> Artificial Sequence

<220>

<223> Chimeric oligonucleotide primer to amplify a portion of human beta-globin gene. “nucleotides 21 to 23 are ribonucleotides-other nucleotides are deoxyribonucleotides, and 5’ end is labeled by biotin”

<400> 41

ctttcttgcc atgagccttc acc 23

<210> 42

<211> 22

<212> DNA

<213> Artificial Sequence

<220>

<223> Chimeric oligonucleotide primer to amplify a portion of human beta-globin gene. “nucleotides 20 to 22 are ribonucleotides-other nucleotides are deoxyribonucleotides, and 5’ end is labeled by biotin”

<400> 42

gccatgagcc ttcaccttag gg 22

<210> 43

<211> 15

<212> DNA

<213> Artificial Sequence

<220>

<223> Oligonucleotide primer for detecting a portion of human beta-globin gene. “5’ end is labeled by FITC”

<400> 43

taacagcatc aggag 15

<210> 44

<211> 13

<212> DNA

<213> Artificial Sequence

<220>

<223> Oligonucleotide primer for detecting a portion of human beta-golbin gene. “5’ end is labeled by FITC”

<400> 44

tcctttgggg atc 13